(19) Europäisches Patentamt
European Patent Office
Office européen des brevets

(11) EP 4 365 287 A1

(12) EUROPEAN PATENT APPLICATION
published in accordance with Art. 153(4) EPC

(43) Date of publication:
**08.05.2024 Bulletin 2024/19**

(21) Application number: **22811391.6**

(22) Date of filing: **27.05.2022**

(51) International Patent Classification (IPC):
***C12N 9/50*** (2006.01) ***C11D 1/28*** (2006.01)
***C11D 1/66*** (2006.01) ***C11D 3/386*** (2006.01)
***C12N 9/20*** (2006.01) ***C12N 9/26*** (2006.01)

(52) Cooperative Patent Classification (CPC):
**C11D 1/28; C11D 1/66; C11D 3/386; C12N 9/20; C12N 9/2408; C12N 9/50**

(86) International application number:
**PCT/JP2022/021663**

(87) International publication number:
**WO 2022/250123 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 JP 2021090160**
**16.09.2021 JP 2021151338**
**28.04.2022 JP 2022074382**

(71) Applicant: **Kao Corporation**
**Tokyo 103-8210 (JP)**

(72) Inventors:
- **KONDO Kensuke**
  **Wakayama-shi, Wakayama 640-8580 (JP)**
- **AONO Keita**
  **Wakayama-shi, Wakayama 640-8580 (JP)**
- **TASE HIRATSUKA Emi**
  **Wakayama-shi, Wakayama 640-8580 (JP)**
- **YAMADA Kozo**
  **Wakayama-shi, Wakayama 640-8580 (JP)**

(74) Representative: **Hoffmann Eitle**
**Patent- und Rechtsanwälte PartmbB**
**Arabellastraße 30**
**81925 München (DE)**

# (54) METHOD FOR PROMOTING ENZYMATIC REACTION

(57) The present invention is a method for accelerating an enzyme reaction including, reacting a substrate with an enzyme in the presence of (a) a sulfosuccinic acid branched alkyl ester having a branched alkyl group with 9 or more and 12 or less carbons or a salt thereof.

EP 4 365 287 A1

## Description

### Field of the Invention

The present invention relates to a method for accelerating an enzyme reaction, an enzyme reaction accelerating agent, an enzyme reaction accelerating agent composition and a detergent composition for use in a dishwasher.

### Background of the Invention

Enzymes such as lipase, amylase, protease or the like are used in many industrial fields such as washing, food processing, paper making or the like.

JP-A S63-7789 discloses a method for producing a fatty acid including, treating oils and fats with lipase in a reversed micelle system composed of an organic solvent, a surfactant and a buffer solution or water.

JP-A 2012-75378 discloses an enzymatic saccharification method for woody biomass including, powdering step (1) of grinding at least one woody biomass selected from a wood chip and a scrap building material to obtain a woody biomass powder, and a saccharifying step (2) of hydrolyzing the woody biomass powder in the presence of a predetermined anionic surfactant and hydrolase to obtain sugar.

Enzymes are utilized in many washing fields.

JP-A 2019-182911 discloses a liquid detergent composition for use in tableware and/or a hard article around a kitchen containing, under predetermined conditions, (a) a sulfosuccinic acid alkyl ester having an alkyl group with 5 or more and 18 or less carbons or a salt thereof, (b) one or more surfactants selected from a semipolar surfactant and an amphoteric surfactant, (c) an enzyme and water.

JP-A 2020-100745 discloses a liquid detergent composition for use in tableware containing, an anionic surfactant of component (A) including one or more components (a1) selected from a secondary alkane sulfonate, a dialkyl sulfosuccinate and a compound represented by a specific formula, an amine oxide-type surfactant of component (B), protease of component (C) and amylase of component (D), wherein a mass ratio of component (A) to component (B) is 0.7 to 2.

Further, in recent years, with the spread of automatic dishwashers, many detergent compositions for use in dishwashers have been proposed. Examples of soil adhering to tableware can include, for example, oil soil, starch soil of rice grains or the like, protein soil of eggs or the like, pigment soil of tea or the like and others. Among these, oil soil or starch soil is soil particularly difficult to remove, and re-adhesion of the soil to tableware to cause fogging of glass tableware or the like is a problem in washing with a dishwasher. Therefore, various formulations have been proposed to increase washing power and anti-re-adhesion performance for the above soil, for example, by combining types of surfactants or enzymes, which are active components for washing, chelating agents and others.

JP-A H4-72397 discloses a technology of formulating specific nonionic surfactant and calcium scavenging chelating agent with alkaline pullulanase having $\alpha$-amylase activity and lipase to allow high washing power to be exhibited. Further, JP-A 2006-152287 describes a technology of combining a high formulation of a chelating agent with a low-foaming nonionic surfactant, thereby preventing fogging of glass tableware to improve finishing performance.

The above JP-A 2019-182911 discloses a liquid detergent composition containing a salt of a sulfosuccinic acid alkyl ester and an enzyme, and a washing method including bringing the composition in the state of foam into direct contact with tableware adhered with oil and fat soil. The above JP-A 2020-100745 discloses that a composition containing a dialkyl sulfosuccinate, protease and amylase is excellent in washing performance and defoaming performance.

### Summary of the Invention

When enzymes are utilized in various industrial fields, they are often reacted with substrates in aqueous media, and hence, reaction fields where water/substrate/enzyme are brought into contact are required. However, as it is often the case that enzymes are hydrophilic while substrates are hydrophobic, there arises a problem that affinities of enzymes for substrates are low, enzyme reactions are less likely to proceed and expected enzyme activity is not obtained. In order to solve this problem, for example, use of large amounts of aqueous solvents, a combined use of surfactants or the like are practiced, but the former is uneconomical as aqueous solvents unnecessary for the reactions are used, and the latter causes a phenomenon that excessive action of surfactants on enzymes reduces enzyme activity itself, though it can improve affinities of enzymes for substrates. Accordingly, there is a strong need for a technology for accelerating the action of substrates and enzymes while maintaining high enzyme activity in aqueous media.

The present invention provides a method for accelerating an enzyme reaction, for example, an enzyme reaction in an aqueous medium.

It is further desirable that dishwasher detergents emulsify oil adhering to tableware with very small amounts of surfactants to prevent washing power from being impaired by foam overflowing into the chamber, but because of small amounts of surfactants, there has not been found yet a technology that attains both high washing power and improved

finishing performance due to prevention of re-adhesion of soil to tableware.

Thus, in dishwasher detergents, a technology that attains both high washing power and prevention of re-adhesion to tableware by using very small amounts of surfactants has been desired, but conventional technologies are not effective enough in preventing oil soil or protein soil from re-adhering to tableware, thus causing white spots on the surface of tableware after washing/drying, leading to significantly impaired finishing performance.

As a cause for this, the present inventors found out a phenomenon that, in a drainage process during the transition from washing to rinsing, some of the oil droplets removed from tableware remain along a circulation line, which is specific to a dishwasher, and at the time of being brought back into the chamber in a rinsing process, oil droplets that have become unstable due to dilution (a decrease in the surfactant concentration), a temperature change (from a high temperature to normal temperature), an agitation stoppage at the time of drainage or the like are combined to increase their particle size, thereby becoming more likely to re-adhere to tableware. Development of an oil soil dispersion stabilization technology also considering this rinsing process specific to a dishwasher has been desired.

The present invention provides a detergent composition for use in a dishwasher excellent in washing power and excellent in finishing performance for tableware. Particularly, the present invention provides a detergent composition for use in a dishwasher excellent in washing power for soil difficult to remove such as oil, starch or the like and excellent in finishing performance for tableware made of plastic or glass.

The present invention relates to a method for accelerating an enzyme reaction including, reacting a substrate with an enzyme in the presence of (a) a sulfosuccinic acid branched alkyl ester having a branched alkyl group with 9 or more and 12 or less carbons or a salt thereof.

Further, the present invention relates to an enzyme reaction accelerating agent containing, (a) a sulfosuccinic acid branched alkyl ester having a branched alkyl group with 9 or more and 12 or less carbons or a salt thereof as an active component.

Further, the present invention relates to an enzyme reaction accelerating agent composition containing, an enzyme reaction accelerating agent containing (a) a sulfosuccinic acid branched alkyl ester having a branched alkyl group with 9 or more and 12 or less carbons or a salt thereof [hereinafter referred to as component (a)] as an active component, and optionally, a surfactant other than component (a) [hereinafter referred to as component (b)], wherein when the composition contains component (b), a mass ratio of a content of component (a) to a content of component (b), (a)/(b), is 0.001 or more and 50 or less.

Further, the present invention relates to a detergent composition for use in a dishwasher containing, (a) a sulfosuccinic acid branched alkyl ester having a branched alkyl group with 9 or more and 12 or less carbons or a salt thereof [hereinafter referred to as component (a)], and (d) an enzyme [hereinafter referred to as component (d)].

According to the present invention, a method for accelerating an enzyme reaction, for example, an enzyme reaction in an aqueous medium is provided.

Further, according to the present invention, a detergent composition for use in a dishwasher excellent in washing power and excellent in finishing performance for tableware can be provided. Particularly, according to the present invention, a detergent composition for use in a dishwasher excellent in washing power for soil difficult to remove such as oil, starch or the like and excellent in finishing performance for tableware made of plastic or glass can be provided.

## Embodiments of the Invention

### [Method for accelerating enzyme reaction]

In the method for accelerating an enzyme reaction of the present invention, a substrate is reacted with an enzyme in the presence of component (a).

In the method for accelerating an enzyme reaction of the present invention, the substrate is preferably reacted with the enzyme in the presence of component (a) and water.

A sulfosuccinic acid ester or a salt thereof is known as a formulation component of a detergent composition for use in a hard article as described, for example, in patent literature 3. However, it has not been known in the art that a compound of a structure qualified to be component (a) of the present invention can significantly accelerate enzyme reactions of various enzymes such as protease or the like. For example, as demonstrated by the comparative examples described later, a compound having a branched alkyl group with 8 carbons does not have an excellent enzyme reaction improvement effect as component (a) of the present invention has even though being analogous in structure to component (a). In other words, the effect of component (a) on enzymes is significantly different from that of even a compound analogous in structure to component (a), and this is an unexpected effect unpredictable to those skilled in the art. Enzymes are used in various industrial fields such as a washing field or the like, and in such a case, they are often used in combination with other agents. The present inventors believe that the ability to accelerate enzyme reactions to improve their effects leads to, for example, a reduction in the use amounts of surfactants, solvents or the like, which is also contributable to the realization of a sustainable society in terms of reducing $CO_2$ emissions.

The mechanism by which the present invention accelerates an enzyme reaction is not limited to the above, but it is inferred to be as follows. In the presence of component (a), as extended wetting of a reaction substrate proceeds in a highly efficient manner, the reactivity of the substrate with an enzyme contained in water can be accelerated. According to the present invention, as use of other surfactants or solvents which causes decreased enzyme activity can be reduced to a minimum, enzymes can maintain their catalytic reactions while retaining high enzyme activity.
Component (a) is preferably a diester. In other words, component (a) is preferably a sulfosuccinic acid di-branched alkyl ester or a salt thereof.
Component (a) is preferably a sulfosuccinic acid branched alkyl ester having a branched alkyl group with 9 or 10 carbons or a salt thereof.
Component (a) is preferably a sulfosuccinic acid branched alkyl ester having a branched alkyl group with 10 carbons or a salt thereof.
Component (a) is preferably a sulfosuccinic acid di-branched alkyl ester or a salt thereof, wherein each of the two branched alkyl groups is a branched alkyl group with 9 or 10 carbons.
Component (a) is preferably a sulfosuccinic acid di-branched alkyl ester or a salt thereof, wherein each of the two branched alkyl groups is a branched alkyl group with 10 carbons.
The method for accelerating an enzyme reaction of the present invention encompasses methods for accelerating an enzyme reaction, wherein these preferable components (a) and the matters stated below are combined.
When component (a) is a salt, examples of the salt include, for example, an alkali metal salt, an alkanolamine salt and others. The salt of component (a) is preferably an alkali metal salt or an alkanolamine salt, more preferably a salt selected from a sodium salt, a potassium salt, a triethanolamine salt, a diethanolamine salt and a monoethanolamine salt and further preferably a sodium salt.
Examples of component (a) include a compound represented by the following formula 1:

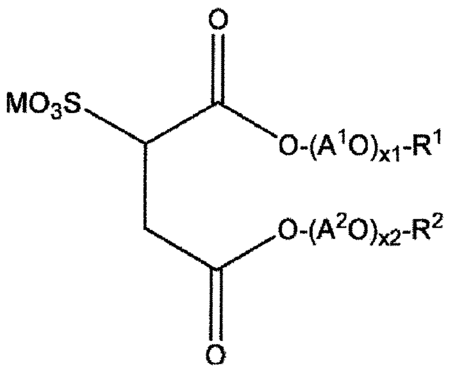

Formula 1

wherein $R^1$ and $R^2$ each represent a branched alkyl group with 9 or more and 12 or less carbons, $A^1O$ and $A^2O$ each represent an alkyleneoxy group with 2 or more and 4 or less carbons, x1 and x2 each represent an average number of added moles, which is a number of 0 or more and 10 or less, and M is a cation.
$R^1$ and $R^2$ may have the same number or different numbers of carbons.

In the present invention, a hydrocarbon residue left over after the removal of a hydroxyl group from a secondary alcohol is included in an open-chain branched hydrocarbon group.
In the present invention, in each of the open-chain branched hydrocarbon groups of $R^1$ and $R^2$, a hydrocarbon chain whose carbon number is the largest when counted from the carbon atom bonded to an oxygen atom is referred to as a main chain, and a hydrocarbon chain branching off from and bonded to the main chain is referred to as a side chain.
When there are two or more possible main chains, in other words, when there are two or more hydrocarbon chains having the largest number of carbons (hereinafter also referred to as the longest hydrocarbon chains), the main chain is determined in the following order:

1. a longest hydrocarbon chain from which a side chain having a larger number of carbon atoms branches off is referred to as the main chain;
2. next, when side chains branching off from the longest hydrocarbon chains have the same number of carbon atoms, a longest hydrocarbon chain from which a larger number of side chains branch off is referred to as the main chain;
3. next, when the same number of side chains branches off from the longest hydrocarbon chains, a longest hydrocarbon chain having a side chain at a carbon atom which is closer to an oxygen atom when counted from the carbon

atom bonded to the oxygen atom is referred to as the main chain; and

4. next, when the carbon atoms having a side chain closest to the oxygen atom are in the same position, a longest hydrocarbon chain having a larger number of carbon atoms in the side chain closest to the oxygen atom is referred to as the main chain.

Note that when there are two or more longest hydrocarbon chains having the same symmetric structure, any of them may be referred to as the main chain.

$R^1$ and $R^2$ in the formula 1 each represent preferably a branched alkyl group selected from a branched nonyl group, a branched decyl group and a branched dodecyl group and more preferably a branched decyl group. The branched decyl group is preferably 2-propylheptyl group.

$A^1O$ and $A^2O$ in the formula 1 each represent an alkyleneoxy group with 2 or more and 4 or less carbons and preferably with 2 or 3 carbons from the viewpoint of lubricity to water. x1 and x2 in the formula 1 represent the average numbers of added moles of $A^1O$ and $A^2O$, and each represent a number of 0 or more and 10 or less, and from the viewpoint of lubricity to water, preferably 6 or less, more preferably 4 or less and further preferably 2 or less, and further preferably 0.

M in the formula 1 is a cation. M is preferably a cation other than a hydrogen ion. Examples of M include, for example, an alkali metal ion such as a lithium ion, a sodium ion, a potassium ion or the like, an alkaline earth metal ion such as a calcium ion, a barium ion or the like, an organic ammonium ion such as a triethanolammonium ion, a diethanolammonium ion, a monoethanolammonium ion, a trimethylammonium ion, a monomethylammonium ion or the like and others.

M is preferably an alkali metal ion or an alkanolammonium ion, more preferably a sodium ion, a potassium ion, a triethanolammonium ion, a diethanolammonium ion or a monoethanolammonium ion and further preferably a sodium ion from the viewpoint of dispersibility in water.

Component (a) of the present invention is preferably a compound represented by the formula 1-1 below. A compound of the formula 1-1 is a compound of the formula 1 in which x1 and x2 each represent 0.

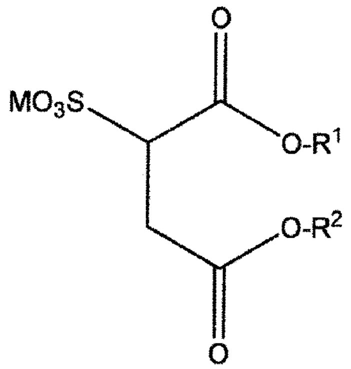

Formula 1-1

wherein $R^1$ and $R^2$ each represent a branched alkyl group with 9 or more and 12 or less carbons, and M is a cation.

The specific examples or preferable examples of $R^1$, $R^2$ and M in the formula 1-1 are the same as in the formula 1.

In the formula 1 or the formula 1-1, a compound in which $R^1$ and $R^2$ are the same is produced by any preparation method not particularly limited, but can be produced by referring to, for example, a method described in US-B 2028091, and an asymmetric compound in which $R^1$ and $R^2$ are different can be produced by referring to, for example, a preparation method in JP-A S58-24555. As a raw material of component (a), a compound with an alkylene oxide added to an alcohol with a predetermined number of carbons can also be used.

Examples of a suitable alcohol used for producing component (a) of the present invention include (1) a primary alcohol represented by 3,5,5-trimethylhexan-1-ol, 2-propylheptan-1-ol or the like and (2) a secondary alcohol represented by 5-nonanol, 2,6-dimethyl-4-heptanol or the like.

In the present invention, an aqueous medium containing component (a), the enzyme and water (hereinafter also referred to as an aqueous medium of the present invention) is preferably brought into contact with the substrate to react the substrate with the enzyme. The aqueous medium of the present invention may be prepared by mixing component (a), the enzyme and water.

A content of component (a) in the aqueous medium of the present invention is preferably 0.001 mass% or more, more preferably 0.01 mass% or more and further preferably 0.05 mass%, and preferably 10 mass% or less, more preferably 5 mass% or less, further preferably 2 mass% or less and furthermore preferably 1 mass% or less.

The aqueous medium of the present invention can optionally contain a surfactant other than component (a) [hereinafter referred to as component (b)], but a content thereof is preferably smaller from the viewpoint of maintaining an enzyme reaction acceleration effect of component (a). A content of component (b) in the aqueous medium of the present invention is preferably 10 mass% or less, more preferably 5 mass% or less, further preferably 3 mass% or less and furthermore preferably 1 mass% or less. The aqueous medium of the present invention may be free of component (b). Note that, when the aqueous medium of the present invention is applied to the substrate in the form of foam or the like, the enzyme may sometimes exhibit its effects efficiently as adhesion efficiency of the enzyme with improved activity is increased. Therefore, when a surfactant for adjusting foaming performance or foam properties of the aqueous medium is used in combination, a content of component (b) in the aqueous medium of the present invention may be outside the above range.

Component (b) is preferably a surfactant less likely to inhibit an enzyme reaction. While optimum component (b) can be appropriately selected in consideration of the embodiments of the method for accelerating an enzyme reaction of the present invention, examples include, for example, one or more selected from (b1) a semipolar surfactant [hereinafter referred to as component (b1)], (b2) an amphoteric surfactant [hereinafter referred to as component (b2)], (b3) an anionic surfactant [hereinafter referred to as component (b3)] and (b4) a nonionic surfactant [hereinafter referred to as component (b4)].

Examples of component (b1) include an amine oxide-type surfactant having one or more and preferably one alkyl group with 8 or more and 14 or less carbons.

Examples of component (b2) include a sulfobetaine-type surfactant having one or more and preferably one alkyl group with 8 or more and 14 or less carbons and a carbobetaine-type surfactant having one or more and preferably one alkyl group with 8 or more and 14 or less carbons.

Examples of component (b3) include an alkyl sulfate having an alkyl group with 8 or more and 14 or less carbons, an alkylbenzene sulfonate having an alkyl group with 8 or more and 14 or less carbons, a polyoxyalkylene alkyl ether sulfate having an alkyl group with 8 or more and 14 or less carbons (in which an oxyalkylene has 2 or 3 carbons and is preferably oxyethylene, and the average number of added moles of an oxyalkylene is 0.5 or more and 5 or less and preferably 0.5 or more and 3 or less) and a fatty acid soap with 8 or more and 14 or less carbons.

Examples of component (b4) include a polyoxyalkylene alkyl ether, for example, a polyoxyalkylene alkyl ether having an alkyl group with 8 or more and 14 or less carbons (in which an oxyalkylene has 2 or 3 carbons and is preferably oxyethylene, and the average number of added moles of an oxyalkylene is 3 or more and 50 or less and preferably 3 or more and 20 or less), an alkyl glycoside, for example, an alkyl glycoside having an alkyl group with 8 or more and 14 or less carbons (in which the average degree of condensation of a sugar backbone of glucose or the like is 1 or more and 5 or less and preferably 1 or more and 2 or less), an alkyl glyceryl ether, for example, an alkyl glyceryl ether having an alkyl group with 8 or more and 12 or less carbons and others.

When the aqueous medium of the present invention contains component (b), a mass ratio of a content of component (a) to a content of component (b), (a)/(b), is preferably 0.001 or more and 50 or less. (a)/(b) can be selected from, for example, further 0.01 or more and 40 or less.

Component (b) is preferably used in an amount falling within the range that the total amount with component (a) does not affect enzyme activity. In the present invention, for example, a mass ratio of a total mass of components (a) and (b) to a total mass of the enzyme, [component (a) + component (b)]/enzyme, is preferably 0.1 or more and more preferably 1 or more, and 200 or less, preferably less than 100, more preferably 50 or less and further preferably 25 or less.

The aqueous medium of the present invention contains water. A content of water in the aqueous medium of the present invention is preferably 50 mass% or more and more preferably 70 mass% or more. Water is used as the balance of the aqueous medium.

The aqueous medium of the present invention can contain a component other than component (a), the enzyme, water and optional component (b). It can contain, for example, a solvent, a hydrotropic agent or the like.

Examples of the solvent [hereinafter referred to as component (c)] can include (c1) a monohydric alcohol with 1 or more and 3 or less carbons, (c2) a polyhydric alcohol with 2 or more and 4 or less carbons, (c3) a di or trialkylene glycol with an alkylene glycol unit having 2 to 4 carbons and (c4) a monoalkoxy (methoxy, ethoxy, propoxy or butoxy), phenoxy or benzoxy ether of a di to tetraalkylene glycol with an alkylene glycol unit having 2 to 4 carbons.

Component (c) is preferably a water-soluble organic solvent with 2 or more and preferably 3 or more, and 10 or less and preferably 8 or less carbons. Here, a water-soluble organic solvent refers to a solvent with an octanol/water partition coefficient (LogPow) of 3.5 or less.

Specific examples of component (c) include ethanol and isopropyl alcohol as (c1), ethylene glycol, propylene glycol, glycerin and isoprene glycol as (c2), diethylene glycol and dipropylene glycol as (c3) and propylene glycol monomethyl ether, propylene glycol monoethyl ether, diethylene glycol monobutyl ether (which is also referred to as butyldiglycol or the like), phenoxy ethanol, phenoxy triethylene glycol and phenoxy isopropanol as (c4). Component (c) is preferably a solvent selected from ethanol, propylene glycol, dipropylene glycol, diethylene glycol monobutyl ether, phenoxy ethanol, phenyl glycol and phenoxy isopropanol. Component (c) is preferably a compound having an alkoxy

group and preferably includes one or more selected from the above (c4), and the aqueous medium of the present invention more preferably contains diethylene glycol monobutyl ether as component (c).

The aqueous medium of the present invention can contain component (c) in an amount of, for example, 0.1 mass% or more and further 0.2 mass% or more, and 10 mass% or less and further 8 mass%.

The hydrotropic agent is preferably a hydrotropic agent selected from an alkylbenzene sulfonic acid having 1 or more and 3 or less alkyl groups with 1 or more and 3 or less carbons and a salt thereof, and specific examples include a hydrotropic agent selected from toluenesulfonic acid, xylenesulfonic acid and cumensulfonic acid and salts of these. A salt is preferably a sodium salt, a potassium salt or a magnesium salt. The hydrotropic agent is preferably p-toluenesulfonic acid or a salt thereof. The aqueous medium of the present invention can contain a hydrotropic agent in an amount of, for example, 0.1 mass% or more and further 0.2 mass% or more, and 10 mass% or less, further 8 mass%, further preferably 7 mass% or less, furthermore preferably 6 mass% or less and furthermore preferably 5 mass% or less.

Component (a) is preferably used such that a mass ratio of the enzyme to component (a) is 0.001 or more, further 0.02 or more and further 0.05 or more, and 15 or less, further 9 or less, further 5 or less and further 1 or less from the viewpoint of an enzyme reaction acceleration effect. Note that, in the present invention, the amount of the enzyme is expressed in terms of the amount of enzyme protein.

Examples of the enzyme include protease, amylase and lipase. The enzyme is preferably one or more selected from protease, amylase and lipase.

The lipase is preferably triacylglycerol lipase on E.C.3.1.1.3, cholesterol esterase on E.C.3.1.1.13, monoacylglycerol lipase on E.C.3.1.23 or lipoprotein lipase on E.C.3.1.1.34. While the origin of the lipase is not limited, examples include lipase of animal origin, plant origin or microbial origin. Examples of lipase of microbial origin include those originated in *Rhizopus, Aspergillus, Mucor, Pseudomonas, Geotrichum, Penicillium, Candida* and others.

As the lipase, Lipase A "AMANO" 6, Lipase AY "AMANO" 30SD, Lipase GS "AMANO" 250G, Lipase R "AMANO," Lipase DF "AMANO" 15 and Lipase MER "AMANO" (which are manufactured by Amano Enzyme Inc.), O lipase (NAGASE & CO., LTD.), Lipase MY, Lipase OF, Lipase PL, Lipase PLC, Lipase QLM, Lipase QLC and Phospholipase D (which are manufactured by Meito Sangyo Co., Ltd.), Lipoprotein lipase (manufactured by Oriental Yeast Co., Ltd.), Lipase (manufactured by Toyo Jozo Co. Ltd.), Lipex, Lipolase and Lipase SP-225 (which are manufactured by Novozymes A/S), Lipase (manufactured by Gist-Brocades International B.V.), and Lipase A and Lipase B (which are manufactured by Sapporo Breweries Ltd.) can be used.

In the present invention, Lipex or Lipolase (both manufactured by Novozymes A/S) is suitable.

Examples of the protease include protease which can act in a neutral or alkaline aqueous solution. Specific examples of a preferable protease include an alkaline protease described in WO-A 99/018218 in which an amino acid sequence shown in Sequence No. 1 or 2 is preferably conserved in an amount of 70% or more, an alkaline protease described in JP-A H5-25492 which is preferably alkaline protease K-16 or alkaline protease K-14 and others. Besides them, examples include the proteases produced by *Bacillus subtilis* sold under the trade names of Savinase®, Kannase®, Everlase®, Alcalase®, Polarzyme® and Esperase® manufactured by Novozymes A/S, the proteases supplied under the trade names of FN2®, FN3®, FN4®, Purafect® and Purafect Prime® manufactured by DuPont de Nemours, Inc. or variants of these and others. Among them, an enzyme described in WO-A 99/018218 in which an amino acid sequence shown in Sequence No. 1 or 2 is conserved in an amount of 80% or more, Savinase, Everlase, Alcalase and Progress manufactured by Novozymes A/S (manufactured by Novozymes A/S) and Purafect and Purafect Prime manufactured by DuPont de Nemours, Inc. are more preferable.

As the amylase, those obtained from many organisms such as bacteria such as *Bacillus subtilis* Marburg, *Bacillus subtilis* natto, *Bacillus amyloliquefaciens, Bacillus licheniformis, Bacillus cereus, Bacillus macerans, Pseudomonas stutzeri, Klebsiella aerogenes* or the like, actinomycetes such as *Streptomyces griseus* or the like, fungi such as *Aspergillus oryzae, Aspergillus niger* or the like, seeds of gramineous and leguminous plants, digestive glands of animals such as humans and pigs or the like and others can be used. Amylase used in the present invention can be obtained in conformity to a general enzyme collection and purification method after inoculating a microorganism as above or a mutant thereof or a host cell transformed with a recombinant vector having a DNA sequence encoding an enzyme therefrom or a mutant thereof or the like into a medium containing assimilable carbon source, nitrogen source and other essential nutrients, and culturing it in the usual manner. While an enzyme solution thus obtained can be used as-is, it can be further purified, crystallized, formulated into powder or formulated into liquid by a publicly-known method and then used. Amylase used in the present invention is preferably $\alpha$-amylase. Examples of commercially available amylases that can be used can include the brand name Rapidase (manufactured by Gist-Brocades International B.V.), the brand names Termamyl, Duramyl and Stainzyme (manufactured by Novozymes Japan Ltd.), Amplify (manufactured by Novozymes A/S) and the brand names Purastar ST and Purastar OxAm (manufactured by Genencor International, Inc.).

The above enzymes can be used as component (d) of the detergent composition for use in a dishwasher of the present invention described later.

The substrate can be selected according to the enzyme, but is preferably one or more selected from protein,

starch and lipid.

Reaction conditions for the substrate and the enzyme can be appropriately set in consideration of the amount of the substrate, enzyme activity or the like. For example, the temperature, pH, composition, use amount, contact time with the substrate or the like of the aqueous medium of the present invention can be set in consideration of the optimum temperature, optimum pH, enzyme activity or the like of the enzyme.

While conditions when the aqueous medium of the present invention is brought into contact with the substrate are not limited, the temperature of the aqueous medium of the present invention can be selected from, for example, 0°C or more, further 10°C or more, further 30°C or more and further 35°C or more, and 100°C or less, further 80°C or less, further 70°C or less and further 50°C or less. Further, the contact time of the aqueous medium of the present invention with the substrate can be selected from, for example, 0.5 minutes or more and further 1.0 minutes or more.

The present invention can be directed to, for example, hydrolysis reactions including a hydrolysis reaction of protein by protease, a hydrolysis reaction of starch by amylase, a hydrolysis reaction of lipid by lipase and a combination of these.

In the present invention, the aqueous medium of the present invention is preferably brought into contact with the substrate in the form of foam from the viewpoint of increasing adhesion efficiency of the enzyme with improved activity to allow the enzyme to exhibit its effects efficiently. In other words, in the present invention, foam obtained by foaming the aqueous medium of the present invention is preferably brought into contact with the substrate.

Further, two or more enzymes can also be used in consideration of the fact that substrates such as protein, starch and lipid or the like are often present in a complexed state in the substrates present in the environment. When two or more enzymes are used, they may be reacted with the substrate separately or they may be combined into the state of a complex enzyme and reacted with the substrate. In either case, sufficient attention should be paid to deactivation due to reactions between enzymes. Further, for a complexed substrate, multiple enzymes with different reactivity toward the substrate can also be acted in a stepwise manner. A combination of these methods is also possible.

As one example, when the enzyme includes lipase, the aqueous medium of the present invention may be an aqueous medium containing component (a) in an amount of 0.01 mass% or more and further 0.1 mass% or more, and 2.0 mass% or less and further 1.0 mass% or less, lipase in an amount of 0.005 mass% or more and further 0.01 mass% or more, and 1.0 mass% or less and further 0.5 mass% or less and water.

As another example, when the enzyme includes protease, the aqueous medium of the present invention may be an aqueous medium containing component (a) in an amount of 0.001 mass% or more and further 0.005 mass% or more, and 5.0 mass%, further 1.0 mass%, further 0.1 mass% or less and further 0.05 mass% or less, protease in an amount of 0.00001 mass% or more and further 0.0001 mass% or more, and 1.0 mass% or less, further 0.5 mass% or less, further 0.1 mass% or less and further 0.01 mass% or less and water.

The aqueous medium of the present invention has a hardness of preferably 0°DH or more and more preferably 4°DH or more, and preferably 20°DH or less, more preferably 10°DH or less and further preferably 8°DH or less in terms of German hardness from the viewpoint of an enzyme reaction acceleration effect. However, when a content of component (a) in the aqueous medium of the present invention is a low concentration, for example, 0.02 mass% or less, the aqueous medium of the present invention preferably has a lower hardness, for example, a hardness of preferably 0°DH as the presence of hardness components has a significant effect on a loss of the active amount of component (a) for accelerating extended wetting of the reaction substrate.

In the present invention, the substrate may be present in soil adhering to an article.

In the present invention, the substrate may be present in soil adhering to a hard surface of an article having the hard surface. Further, in the present invention, the substrate may be present in soil adhering to fibers (clothing or the like).

When the substrate is present in soil adhering to an article, for example, an article having a hard surface or fibers, bringing the aqueous medium of the present invention into contact with the soil accelerates an enzyme reaction in situ. It is considered that the method of the present invention also improves a soil removal effect as substrates such as protein, starch and lipid or the like are often soil itself.

One example of the method for accelerating an enzyme reaction of the present invention is a method for accelerating an enzyme reaction including, reacting lipid with lipase in the presence of component (a).

Another example of the method for accelerating an enzyme reaction of the present invention is a method for accelerating an enzyme reaction including, bringing an aqueous medium containing component (a), lipase and water into contact with lipid to react the lipid with the lipase.

[Enzyme reaction accelerating agent and enzyme reaction accelerating agent composition]

The present invention is based on the finding that an enzyme reaction is accelerated by component (a).

The present invention relates to an enzyme reaction accelerating agent containing component (a) as an active component.

The enzyme reaction accelerating agent of the present invention may be an enzyme reaction accelerating agent substantially composed of component (a).

The present invention relates to an enzyme reaction accelerating agent composition containing component (a) and optionally component (b), wherein when the composition contains component (b), a mass ratio of a content of component (a) to a content of component (b), (a)/(b), is 0.001 or more and 50 or less.

The present invention relates to use of component (a) as an enzyme reaction accelerating agent.

The present invention relates to use as an enzyme reaction accelerating agent composition of a composition containing component (a) and optionally component (b), wherein when the composition contains component (b), a mass ratio of a content of component (a) to a content of component (b), (a)/(b), is 0.001 or more and 50 or less.

The matters stated in the method for accelerating an enzyme reaction of the present invention can be appropriately applied to each of the enzyme reaction accelerating agent, the enzyme reaction accelerating agent composition and the use of the present invention. The specific examples or preferable examples of component (a), component (b), the enzyme, the substrate or the like in the enzyme reaction accelerating agent, the enzyme reaction accelerating agent composition and the use of the present invention are also the same as in the method for accelerating an enzyme reaction of the present invention. The above mass ratio (a)/(b) can be selected from, for example, 0.001 or more and further 0.01 or more, and preferably 50 or less and further 40 or less.

[Detergent composition for use in dishwasher]

The detergent composition for use in a dishwasher of the present invention contains (a) a sulfosuccinic acid branched alkyl ester having a branched alkyl group with 9 or more and 12 or less carbons [hereinafter referred to as component (a)], and (d) an enzyme [hereinafter referred to as component (d)].

The mechanism by which the detergent composition for use in a dishwasher of the present invention improves washing power and finishing performance for tableware is uncertain, but it is inferred to be as follows.

It is considered that the detergent composition for use in a dishwasher of the present invention exhibits its washing effect due to soil removal performance and re-adhesion prevention owing to components (a) and (d) contained therein. Note that the acting mechanism of the detergent composition for use in a dishwasher of the present invention is not limited thereto.

<Component (a)>

Component (a) is a sulfosuccinic acid branched alkyl ester having a branched alkyl group with 9 or more and 12 or less carbons. The specific examples or preferable aspects of component (a) can be selected from those stated in the method for accelerating an enzyme reaction of the present invention.

In the detergent composition for use in a dishwasher of the present invention, the branched alkyl group of component (a) is preferably a branched alkyl group having a main chain with 6 or 7 carbons and one or more side chains, the side chains having 2 or more and 4 or less carbons in total.

In the detergent composition for use in a dishwasher of the present invention, the branched alkyl group of component (a) is preferably a branched alkyl group selected from 2-propylheptyl group, 4-methyl 2-propylhexyl group, 5-methyl 2-propylhexyl group and 3,5,5-trimethylhexyl group and more preferably a branched alkyl group selected from 2-propylheptyl group and 3,5,5-trimethylhexyl group.

Examples of component (a) of the detergent composition for use in a dishwasher of the present invention include a compound represented by the above formula 1 and further a compound represented by the above formula 1-1.

In the detergent composition for use in a dishwasher of the present invention, side chains in the branched alkyl groups of $R^1$ and $R^2$ in the above formula 1 or formula 1-1 may have the same or different total numbers of constituent carbons, which are preferably 3 from the viewpoint of washing power.

In the present invention, the total number of carbons constituting a side chain is the total number of carbons in all the side chains other than the main chain in one branched alkyl group, and when there is a plurality of side chains, it refers to the total number of carbons in all those side chains.

In the detergent composition for use in a dishwasher of the present invention, $R^1$ and $R^2$ in the above formula 1 or formula 1-1 may have the same number or different numbers of side chains, and each have one or more, and preferably 3 or less and more preferably 2 or less side chains from the viewpoint of washing power.

In the present invention, the number of side chains is the number of side chains branching off from the main chain, and even if a side chain further has a side chain branching off from the side chain, the number of side chains remains the same. However, a side chain may further have a side chain branching off from the side chain from the viewpoint of washing power.

In the detergent composition for use in a dishwasher of the present invention, $R^1$ and $R^2$ in the above formula 1 or formula 1-1 may have the same number or different numbers of branch carbons, and each have one or more, and

preferably 3 or less and furthermore preferably 2 or less branch carbons from the viewpoint of washing power.

In the present invention, the number of branch carbons is the total of the number of tertiary carbon atoms and the number of quaternary carbon atoms in an open-chain branched hydrocarbon group.

In the detergent composition for use in a dishwasher of the present invention, the open-chain branched hydrocarbon groups of $R^1$ and $R^2$ in a preferable aspect of $R^1$ and $R^2$ in the above formula 1 or formula 1-1 each independently have 9 or more and 11 or less carbons and further 9 or 10 carbons in total, each independently have a main chain with 6 or 7 carbons, each independently have a side chain with 1 or more and 3 or less constituent carbons and each independently have one side chain.

In the detergent composition for use in a dishwasher of the present invention, the branched alkyl groups of $R^1$ and $R^2$ in the above formula 1 or formula 1-1 may be the same or different, and specific examples include a branched alkyl group selected from 2-propylheptyl group, 4-methyl 2-propylehexyl group, 5-methyl 2-propylhexyl group and 3,5,5-trimethylhexyl group.

In the detergent composition for use in a dishwasher of the present invention, $A^1O$ and $A^2O$ in the above formula 1 or formula 1-1 each independently represent an alkyleneoxy group with 2 or more, and 4 or less and preferably 3 or less carbons.

In the detergent composition for use in a dishwasher of the present invention, x1 and x2 in the above formula 1 or formula 1-1 are average numbers of added moles and each independently represent 0 or more, and 6 or less, preferably 4 or less, more preferably 2 or less and further preferably 0 from the viewpoint of washing power.

Further, x1 + x2 is preferably 0 or more, and preferably 12 or less, more preferably 6 or less, further preferably 3 or less and furthermore preferably 0 from the viewpoint of washing power.

In the detergent composition for use in a dishwasher of the present invention, M in the above formula 1 or formula 1-1 is a hydrogen ion, or an inorganic cation such as a sodium ion, an ammonium ion, a potassium ion, a magnesium ion or the like or an organic cation such as a monoethanolammonium ion, a diethanolammonium ion, a triethanolammonium ion, a morpholinium ion or the like, and preferably an inorganic cation selected from a sodium ion, an ammonium ion, a potassium ion and a magnesium ion.

<Component (d)>

Component (d) is an enzyme. Examples of component (d) include one or more enzymes selected from amylase, protease and lipase. Component (d) is preferably two or more enzymes selected from amylase, protease and lipase and preferably two or more enzymes including at least amylase and protease from the viewpoint of washing performance. The specific examples or preferable aspects of component (d) can be selected from those stated in the method for accelerating an enzyme reaction of the present invention.

<Composition and other components or the like of detergent composition for use in dishwasher of the present invention>

The detergent composition for use in a dishwasher of the present invention can contain component (a) in an amount of, for example, 0.01 mass% or more, further 0.05 mass% or more and further 0.1 mass% or more from the viewpoint of washing power, and for example, 5 mass% or less, further 3 mass% or less, further 2 mass% or less and further 1 mass% or less from the viewpoint of storage stability. Note that, in the present invention, the description regarding the amount of component (a) (mass% or the like) is based on a mass calculated by assuming that a compound is a sodium salt, for example, a mass given by assuming that M in the above formula 1 is sodium.

The detergent composition for use in a dishwasher of the present invention can contain component (d) in an amount of preferably 0.001 mass% or more, more preferably 0.005 mass% or more and further preferably 0.01 mass% or more, and preferably 15 mass% or less, more preferably 9 mass% or less and further preferably 5 mass% or less in terms of enzyme protein from the viewpoints of washing power and costs. Note that, as a method for quantifying enzyme protein for component (d), the Lowry method can be employed.

A mass ratio of a content of component (a) to a content of component (d) in the detergent composition for use in a dishwasher of the present invention, (a)/(d), is preferably 0.1 or more, more preferably 1 or more and further preferably 10 or more, and preferably 50 or less, more preferably 40 or less and further preferably 30 or less from the viewpoint of washing performance. The content of component (d) in the detergent composition for use in a dishwasher of the present invention is expressed in terms of the mass of enzyme protein.

The detergent composition for use in a dishwasher of the present invention can optionally contain a surfactant other than component (a) [component (b) described earlier]. The specific examples or preferable aspects of component (b) can be selected from those stated in the method for accelerating an enzyme reaction of the present invention. Examples of component (b) include, for example, an amphoteric surfactant [component (b2) described earlier], an anionic surfactant (excluding component (a)) [component (b3) described earlier], a nonionic surfactant [component (b4) described earlier] and others.

A proportion of a content of component (a) in all the surfactants contained in the detergent composition for use in a dishwasher of the present invention may be, for example, 5 mass% or more, further 8 mass% or more and further 10 mass% or more, and for example, 100 mass% or less, further 50 mass% or less and further 30 mass% or less from the viewpoint of washing performance.

When the detergent composition for use in a dishwasher of the present invention contains an anionic surfactant other than component (a), a proportion of a content of component (a) in all the anionic surfactants contained in the detergent composition for use in a dishwasher of the present invention is, for example, 8 mass% or more, preferably 50 mass% or more and more preferably 90 mass% or more, and 100 mass% or less from the viewpoint of attaining both washing power and anti-foaming performance. A proportion of a content of component (a) in all the anionic surfactants may be 100 mass%.

<Component (b4)>

The detergent composition for use in a dishwasher of the present invention can optionally contain (b4) a nonionic surfactant [hereinafter referred to as component (b4)]. Component (b4) is a component preferable from the viewpoints of storage stability and washing power.

In the detergent composition for use in a dishwasher of the present invention, component (b4) is preferably one or more nonionic surfactants selected from an alkyl glycoside, an alkyl glyceryl ether and a polyoxyalkylene alkyl ether.

The alkyl glycoside has an alkyl group with preferably 8 or more, and preferably 14 or less, more preferably 12 or less and further preferably 10 or less carbons.

The alkyl glyceryl ether has an alkyl group with preferably 8 or more, and preferably 14 or less, more preferably 12 or less and further preferably 10 or less carbons.

The polyoxyalkylene alkyl ether has an alkyl group with preferably 8 or more, and preferably 14 or less, more preferably 12 or less and further preferably 10 or less carbons, and the average number of added moles of an oxyalkylene group and more preferably an oxyethylene group is preferably 3 or more, and preferably 20 or less and more preferably 10 or less. Component (b4) is preferably a nonionic surfactant having an above alkyl group, the alkyl group being derived from a secondary alcohol, and is preferably, for example, a polyoxyalkylene alkyl ether with an oxyethylene group added to a secondary alcohol at an average number of added moles of 1 or more and 10 or less. Component (b4) is preferably anti-foaming one.

The detergent composition for use in a dishwasher of the present invention can contain component (b4) in an amount of preferably 0.05 mass% or more, more preferably 0.1 mass% or more and further preferably 0.3 mass% or more from the viewpoints of storage stability and washing power, and preferably 5 mass% or less, more preferably 3 mass% or less and further preferably 1.0 mass% or less from the viewpoint of anti-foaming performance.

The detergent composition for use in a dishwasher of the present invention can contain an anti-foaming agent. Examples of the anti-foaming agent include a high-molecular compound such as silicone, polypropylene glycol or the like, hydrophobic silica particles, and others. The anti-foaming agent may be, for example, an oil compound obtained by formulating silicone oil with hydrophobic silica fine powder. Further, the anti-foaming agent may be selected from compounds functioning as an oil agent or a solvent, and the compounds may be high-molecular compounds or low-molecular compounds. The anti-foaming agent is preferably a high-molecular compound and more preferably polypropylene glycol.

A weight average molecular weight of polypropylene glycol is preferably 1,000 or more, more preferably 2,000 or more and further preferably 3,000 or more from the viewpoint of anti-foaming performance, and preferably 10,000 or less, more preferably 8,000 or less and further preferably 5,000 or less from the viewpoint of formulation stability. Here, a weight average molecular weight of polypropylene glycol is calculated on the basis of measurements of a hydroxyl value (mg KOH/g). Specifically, a weight average molecular weight of polypropylene glycol can be calculated by ($m \times 1000 \times 56/OHV$). Here, m is the number of hydroxyl groups in polypropylene glycol, and m is 2 in the case of polypropylene glycol of a diol-type and m is 3 in the case of that of a triol-type. Further, OHV (mg KOH/g) is a hydroxyl value measured in conformity to JIS K 0070.

The detergent composition for use in a dishwasher of the present invention can contain an anti-foaming agent in an amount of preferably 0.01 mass% or more, more preferably 0.05 mass% or more and further preferably 0.1 mass% or more from the viewpoint of storage stability, and preferably 5 mass% or less, more preferably 4 mass% or less and further preferably 3 mass% or less from the viewpoint of anti-foaming performance.

The detergent composition for use in a dishwasher of the present invention can contain a chelating agent.

As the chelating agent, citric acid, malic acid, tartaric acid, succinic acid, methylglycinediacetic acid, glutamic acid diacetic acid, hydroxyethyl iminodiacetic acid, ethylenediamine disuccinic acid, nitrilotriacetic acid, 1,3-propanediamine triacetic acid, 1,3-diamino-2-hydroxypropane tetraacetic acid, glycol ether diamine tetraacetic acid, ethylenediaminetetraacetic acid, diethylenetriaminepentaacetic acid, triethylenetetramine hexaacetic acid, dihydroxyethylglycine, a polycarboxylic acid such as hydroxyethyl ethylenediamine dicarboxymethyl glutamic acid or the like or salts of these,

or tripolyphosphoric acid or a salt thereof can be used. Examples of a salt of the chelating agent include an alkali metal salt such as a sodium salt, a potassium salt or the like.

The chelating agent is preferably one or more selected from citric acid, malic acid, ethylenediaminetetraacetic acid, methylglycinediacetic acid, glutamic acid diacetic acid, tripolyphosphoric acid and salts of these, and more preferably one or more selected from citric acid, trisodium citrate, glutamic acid diacetic acid and salts of these.

When the detergent composition for use in a dishwasher of the present invention contains a chelating agent, the composition can contain the chelating agent in an amount of, for example, 2 mass% or more, further 5 mass% or more and further 10 mass% or more, and 30 mass% or less, further 25 mass% or less and further 20 mass% or less.

The detergent composition for use in a dishwasher of the present invention can optionally contain a thickener such as xanthan gum or the like, a defoamer, a bleaching agent or the like.

The detergent composition for use in a dishwasher of the present invention may be either a liquid detergent composition or a powder detergent composition.

When the detergent composition for use in a dishwasher of the present invention is liquid, the detergent composition may contain water. Water may be the balance left over after excluding component (a), component (d), component (b4) and other optional components.

Further, when the detergent composition for use in a dishwasher of the present invention is powder, the detergent composition may contain sodium sulfate (sodium sulfate-decahydrate). Sodium sulfate may be the balance left over after excluding component (a), component (d), component (b4) and other optional components.

When the detergent composition for use in a dishwasher of the present invention is liquid, the detergent composition for use in a dishwasher of the present invention has a pH at 25°C of preferably 5 or more, more preferably 6 or more and further preferably 7 or more from the viewpoint of washing performance, and preferably 11 or less, more preferably 10 or less and further preferably 8 or less from the viewpoint of a sense of security at the time of handling. Note that the pH can be measured by a glass electrode method (pH below can also be measured by the same method).

When the detergent composition for use in a dishwasher of the present invention is liquid, the detergent composition for use in a dishwasher of the present invention has a viscosity at 25°C of preferably 10 mPa·s or more, more preferably 50 mPa·s or more and further preferably 100 mPa·s or more from the viewpoint of detergent putting performance, and preferably 5,000 mPa·s or less and more preferably 2,500 mPa·s or less from the viewpoint of suppression of an undissolved detergent residue. This viscosity is a value measured by a B-type viscometer (rotor No. 2, 6 rpm, measurement time 1 minute, liquid temperature 25°C).

When the detergent composition for use in a dishwasher of the present invention is powder, the detergent composition for use in a dishwasher of the present invention may contain an alkali agent. Examples of the alkali agent include sodium carbonate, potassium carbonate, sodium hydrogencarbonate, ethanolamine, sodium silicate, potassium silicate and others.

The detergent composition for use in a dishwasher of the present invention can be used as a washing liquid prepared by diluting it with water (hereinafter referred to as a washing liquid of the present invention) at the time of use. The washing liquid of the present invention may be a mixture containing the detergent composition for use in a dishwasher of the present invention and water.

A concentration at the time of use of the detergent composition for use in a dishwasher of the present invention (dilution concentration) is preferably 0.01 mass% or more, more preferably 0.05 mass% or more and further preferably 0.1 mass% or more from the viewpoint of washing performance, and preferably 2 mass% or less, more preferably 1 mass% or less and further preferably 0.5 mass% or less from the viewpoint of foaming.

A concentration of component (a) at the time of use of the detergent composition for use in a dishwasher of the present invention is preferably 1 ppm or more, more preferably 2 ppm or more and further preferably 5 ppm or more from the viewpoint of washing performance, and preferably 100 ppm or less, more preferably 50 ppm or less and further preferably 30 ppm or less from the same viewpoint.

A concentration of component (d) at the time of use of the detergent composition for use in a dishwasher of the present invention is preferably 0.001 ppm or more, more preferably 0.005 ppm or more and further preferably 0.01 ppm or more from the viewpoint of washing performance, and preferably 5 ppm or less, more preferably 2 ppm or less and further preferably 1 ppm or less from the same viewpoint. The mass of component (d) is expressed in terms of the mass of enzyme protein.

The detergent composition for use in a dishwasher of the present invention can be used as a washing liquid prepared by appropriately diluting it with water or the like at the time of use, and a washing liquid prepared by diluting the detergent composition for use in a dishwasher of the present invention has a pH of preferably 5 or more, more preferably 6 or more and further preferably 6.5 or more, and preferably 12 or less, more preferably 11.5 or less and further preferably 11 or less at 25°C.

For example, a mixture of the detergent composition for use in a dishwasher of the present invention in an amount of 0.2 mass% and water has a pH of preferably 5 or more, more preferably 6 or more and further preferably 6.5 or more, and preferably 12 or less, more preferably 11.5 or less and further preferably 11 or less at 25°C.

[Method for washing tableware]

The detergent composition for use in a dishwasher of the present invention can be used for a dishwasher and further for an automatic dishwasher, and is further suitably used for washing in a dishwasher for business use. Note that tableware includes cooking utensils, washing utensils or the like.

At the time of washing in a dishwasher and further in a dishwasher for business use, the detergent composition for use in a dishwasher of the present invention is used as a washing liquid prepared by mixing it with water. At that time, a certain amount of the composition is arbitrarily transferred to the inside of a dishwasher through a supplying device to maintain an appropriate concentration of the washing liquid.

According to the present invention, provided are a washing liquid prepared by diluting the detergent composition for use in a dishwasher of the present invention with water, and a washing method including, washing tableware in a dishwasher with the washing liquid. This washing method may include a step of preparing a washing liquid by diluting the detergent composition for use in a dishwasher of the present invention with water, and a step of washing tableware in a dishwasher and further in an automatic dishwasher with the washing liquid.

In this step of preparing a washing liquid, the composition is diluted such that the concentrations of the detergent composition for use in a dishwasher of the present invention, component (a) and component (b) in the washing liquid each fall within the above concentration range at the time of use of the detergent composition for use in a dishwasher of the present invention. Further, the pH range of the washing liquid at 25°C is the same as the above preferable pH range at 25°C of the washing liquid prepared by diluting the detergent composition for use in a dishwasher of the present invention.

The washing liquid has a washing temperature of preferably 30°C or more and more preferably 40°C or more from the viewpoints of productivity and washing performance, and preferably 80°C or less and more preferably 70°C or less from the viewpoint of damage to base materials of tableware.

Immediately after washed, tableware is usually rinsed with water, warm water or hot water, which is, for example, hot water at preferably 30°C or more and more preferably 40°C or more, and preferably 80°C or less, for 5 minutes or more and 40 minutes or less in the same dishwasher and further automatic dishwasher.

In a dishwasher, a washing liquid is usually circulated with a pump and repeatedly used.

The detergent composition for use in a dishwasher of the present invention is used for washing tableware in a dishwasher. Examples of tableware include articles used for containing food, such as a dish, a bowl, a cup or the like, articles used for serving food or the like, such as chopsticks, a folk, a knife, a spoon or the like, and others. In the present invention, tableware may mean including, in addition to these articles, articles that come in contact with food and can be washed in a dishwasher, such as storage jars such as Tupperware®, a bottle or the like, cooking utensils such as a kitchen knife, a chopping board, a pan, a frying pan, a fish grill or the like, cooking appliances such as a food processor, a mixer or the like and others, or parts of these. Examples of a material of tableware include plastic (including silicone resin or the like), metal, ceramic, wood and a combination of these. The detergent composition for use in a dishwasher of the present invention is suitably used for washing tableware having a hydrophobic surface, for example, plastic tableware. Washing with the detergent composition for use in a dishwasher of the present invention conforms to usual dishwasher washing.

[Aspect of the present invention]

The following aspects of the present invention are described by way of example. The matters stated in the method for accelerating an enzyme reaction and the detergent composition for use in a dishwasher of the present invention can be applied to these aspects with modifications as necessary. Further, the subject matter of each aspect can be applied to other aspects with modifications as necessary.

<1>

A method for accelerating an enzyme reaction including, reacting a substrate with an enzyme in the presence of (a) a sulfosuccinic acid branched alkyl ester having a branched alkyl group with 9 or more and 12 or less carbons or a salt thereof [hereinafter referred to as component (a)].

<2>

The method for accelerating an enzyme reaction according to <1>, wherein component (a) is a sulfosuccinic acid branched alkyl ester having a branched alkyl group with 9 or 10 carbons or a salt thereof.

<3>

The method for accelerating an enzyme reaction according to <1> or <2>, wherein component (a) is a sulfosuccinic acid di-branched alkyl ester or a salt thereof, wherein each of the two branched alkyl groups is a branched alkyl group with 9 or 10 carbons.

<4>

The method for accelerating an enzyme reaction according to any of <1> to <3>, wherein the branched alkyl group of component (a) is 2-propylheptyl group.

<5>

The method for accelerating an enzyme reaction according to any of <1> to <4>, wherein component (a) is a sulfosuccinic acid branched alkyl ester or a salt thereof represented by the following formula 1:

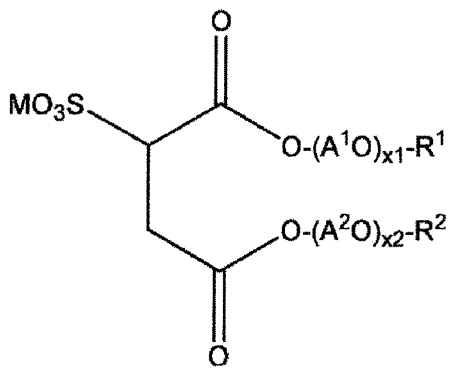

Formula 1

wherein $R^1$ and $R^2$ each represent a branched alkyl group with 9 or more and 12 or less carbons, $A^1O$ and $A^2O$ each represent an alkyleneoxy group with 2 or more and 4 or less carbons, x1 and x2 each represent an average number of added moles, which is a number of 0 or more and 10 or less, and M is a cation.

<6>

The method for accelerating an enzyme reaction according to <5>, wherein $R^1$ and $R^2$ in the formula 1 each represent a branched alkyl group selected from a branched nonyl group, a branched decyl group and a branched dodecyl group and preferably a branched decyl group.

<7>

The method for accelerating an enzyme reaction according to <5> or <6>, wherein $A^1O$ and $A^2O$ in the formula 1 each represent an alkyleneoxy group with 2 or more and 4 or less carbons and preferably with 2 or 3 carbons.

<8>

The method for accelerating an enzyme reaction according to any of <5> to <7>, wherein x1 and x2 in the formula 1 each represent a number of 0 or more, and 10 or less, preferably 6 or less, more preferably 4 or less and further preferably 2 or less, and furthermore preferably 0.

<9>

The method for accelerating an enzyme reaction according to any of <5> to <8>, wherein M in the formula 1 is an alkali metal ion or an alkanolammonium ion, preferably a sodium ion, a potassium ion, a triethanolammonium ion, a diethanolammonium ion or a monoethanolammonium ion and further preferably a sodium ion.

<10>

The method for accelerating an enzyme reaction according to any of <1> to <9>, wherein an aqueous medium containing component (a), the enzyme and water is brought into contact with the substrate to react the substrate with the enzyme.

<11>

The method for accelerating an enzyme reaction according to <10>, wherein a content of component (a) in the aqueous medium is preferably 0.001 mass% or more, more preferably 0.01 mass% or more and further preferably 0.05 mass%, and preferably 10 mass% or less, more preferably 5 mass% or less, further preferably 2 mass% or less and furthermore preferably 1 mass% or less.

<12>

The method for accelerating an enzyme reaction according to <10> or <11>, wherein a content of component (a) in the aqueous medium is 0.01 mass% or more, and 2 mass% or less and further 1 mass% or less.

<13>

The method for accelerating an enzyme reaction according to any of <10> to <12>, wherein the aqueous medium optionally contains a surfactant other than component (a) [hereinafter referred to as component (b)], and a content of component (b) in the aqueous medium is 5.0 mass% or less.

<14>

The method for accelerating an enzyme reaction according to <13>, wherein a content of component (b) in the aqueous medium is 3.0 mass% or less.

<15>

The method for accelerating an enzyme reaction according to <13> or <14>, wherein component (b) is one or more selected from (b1) a semipolar surfactant [hereinafter referred to as component (b1)], (b2) an amphoteric surfactant [hereinafter referred to as component (b2)], (b3) an anionic surfactant [hereinafter referred to as component (b3)] and (b4) a nonionic surfactant [hereinafter referred to as component (b4)].

<16>

The method for accelerating an enzyme reaction according to <15>, wherein component (b1) is an amine oxide-type surfactant having one or more and preferably one alkyl group with 8 or more and 14 or less carbons.

<17>

The method for accelerating an enzyme reaction according to <15> or <16>, wherein component (b2) is an amphoteric surfactant selected from a sulfobetaine-type surfactant having one or more and preferably one alkyl group with 8 or more and 14 or less carbons and a carbobetaine-type surfactant having one or more and preferably one alkyl group with 8 or more and 14 or less carbons.

<18>

The method for accelerating an enzyme reaction according to any of <15> to <17>, wherein component (b3) is an anionic surfactant selected from an alkyl sulfate having an alkyl group with 8 or more and 14 or less carbons, an alkylbenzene sulfonate having an alkyl group with 8 or more and 14 or less carbons, a polyoxyalkylene alkyl ether sulfate having an alkyl group with 8 or more and 14 or less carbons (in which an oxyalkylene has 2 or 3 carbons and is preferably oxyethylene, and the average number of added moles of an oxyalkylene is 0.5 or more and 5 or less and preferably 0.5 or more and 3 or less) and a fatty acid soap with 8 or more and 14 or less carbons.

<19>

The method for accelerating an enzyme reaction according to any of <13> to <18>, wherein the aqueous medium contains component (b), and a mass ratio of a content of component (a) to a content of component (b), (a)/(b), is 0.001 or more and further 0.01 or more, and 50 or less and further 40 or less.

<20>

The method for accelerating an enzyme reaction according to any of <13> to <19>, wherein the aqueous medium contains component (b), and a mass ratio of a total mass of components (a) and (b) to a total mass of the enzyme, [component (a) + component (b)]/enzyme, is preferably 0.1 or more and more preferably 1 or more, and 200 or less, preferably less than 100 and more preferably 50 or less.

<21>

The method for accelerating an enzyme reaction according to any of <10> to <20>, wherein a content of water in the aqueous medium is preferably 50 mass% or more and more preferably 70 mass% or more.

<22> The method for accelerating an enzyme reaction according to any of <10> to <21>, wherein the aqueous medium contains a solvent [hereinafter referred to as component (c)].

<23>

The method for accelerating an enzyme reaction according to <22>, wherein component (c) is one or more selected from (c1) a monohydric alcohol with 1 or more and 3 or less carbons, (c2) a polyhydric alcohol with 2 or more and 4 or less carbons, (c3) a di or trialkylene glycol with an alkylene glycol unit having 2 to 4 carbons and (c4) a monoalkoxy (in which an alkoxy is methoxy, ethoxy, propoxy or butoxy), phenoxy or benzoxy ether of a di to tetraalkylene glycol with an alkylene glycol unit having 2 to 4 carbons.

<24>

The method for accelerating an enzyme reaction according to <22> or <23>, wherein the aqueous medium contains a water-soluble organic solvent with an octanol/water partition coefficient (LogPow) of 3.5 or less as component (c).

<25>

The method for accelerating an enzyme reaction according to any of <22> to <24>, wherein the aqueous medium contains, as component (c), one or more selected from ethanol and isopropyl alcohol of (c1), ethylene glycol, propylene glycol, glycerin and isoprene glycol of (c2), diethylene glycol and dipropylene glycol of (c3) and propylene glycol monomethyl ether, propylene glycol monoethyl ether, diethylene glycol monobutyl ether, phenoxy ethanol, phenoxy triethylene glycol and phenoxy isopropanol of (c4), preferably one or more selected from ethanol, propylene glycol, dipropylene glycol, diethylene glycol monobutyl ether, phenoxy ethanol, phenyl glycol and phenoxy isopropanol and more preferably diethylene glycol monobutyl ether.

<26>

The method for accelerating an enzyme reaction according to any of <22> to <25>, wherein the aqueous medium contains component (c) in an amount of 0.1 mass% or more and further preferably 0.2 mass% or more, and 10 mass% or less and further preferably 8 mass%.

<27>

The method for accelerating an enzyme reaction according to any of <10> to <26>, wherein the aqueous medium contains a hydrotropic agent, preferably one or more hydrotropic agents selected from an alkylbenzene sulfonic acid having 1 or more and 3 or less alkyl groups with 1 or more and 3 or less carbons and a salt thereof and more

preferably one or more hydrotropic agents selected from toluenesulfonic acid, xylenesulfonic acid and cumensulfonic acid and salts of these.

<28>

The method for accelerating an enzyme reaction according to any of <10> to <27>, wherein the aqueous medium is brought into contact with the substrate in the state of foam.

<29>

The method for accelerating an enzyme reaction according to any of <10> to <28>, wherein, when the aqueous medium is brought into contact with the substrate, the temperature of the aqueous medium is selected from 0°C or more, further 10°C or more, further 30°C or more and further 35°C or more, and 100°C or less, further 80°C or less, further 70°C or less and further 50°C or less, and the contact time is selected from 0.5 minutes or more and further 1.0 minutes or more.

<30>

The method for accelerating an enzyme reaction according to any of <10> to <29>, wherein the aqueous medium contains lipase as the enzyme, and the aqueous medium is an aqueous medium containing component (a) in an amount of 0.01 mass% or more and further 0.1 mass% or more, and 2.0 mass% or less and further 1.0 mass% or less, lipase in an amount of 0.005 mass% or more and further 0.01 mass% or more, and 1.0 mass% or less and further 0.5 mass% or less, and water.

<31>

The method for accelerating an enzyme reaction according to any of <10> to <30>, wherein the aqueous medium contains protease as the enzyme, and the aqueous medium is an aqueous medium containing component (a) in an amount of 0.001 mass% or more and further 0.005 mass% or more, and 5.0 mass%, further 1.0 mass%, further 0.1 mass% or less and further 0.05 mass% or less, protease in an amount of 0.00001 mass% or more and further 0.0001 mass% or more, and 1.0 mass% or less, further 0.5 mass% or less, further 0.1 mass% or less and further 0.01 mass% or less, and water.

<32>

The method for accelerating an enzyme reaction according to any of <10> to <31>, wherein the aqueous medium has a hardness of preferably 0°DH or more and more preferably 4°DH or more, and preferably 20°DH or less, more preferably 10°DH or less and further preferably 8°DH or less in terms of German hardness.

<33>

The method for accelerating an enzyme reaction according to any of <1> to <32>, wherein component (a) is used such that a mass ratio of the enzyme to component (a) is 0.001 or more, preferably 0.02 or more and further preferably 0.05 or more, and 15 or less, preferably 9 or less and further preferably 5 or less.

<34>

The method for accelerating an enzyme reaction according to any of <1> to <33>, wherein the enzyme is one or more selected from protease, amylase and lipase.

<35>

The method for accelerating an enzyme reaction according to <34>, wherein the lipase is one or more selected from triacylglycerol lipase on E.C.3.1.1.3, cholesterol esterase on E.C.3.1.1.13, monoacylglycerol lipase on E.C.3.1.23 and lipoprotein lipase on E.C.3.1.1.34.

<36>

The method for accelerating an enzyme reaction according to <34> or <35>, wherein the lipase is one or more selected from Lipase A "AMANO" 6, Lipase AY "AMANO" 30SD, Lipase GS "AMANO" 250G, Lipase R "AMANO," Lipase DF "AMANO" 15 and Lipase MER "AMANO" (which are manufactured by Amano Enzyme Inc.), O lipase (NAGASE & CO., LTD.), Lipase MY, Lipase OF, Lipase PL, Lipase PLC, Lipase QLM, Lipase QLC and Phospholipase D (which are manufactured by Meito Sangyo Co., Ltd.), Lipoprotein lipase (manufactured by Oriental Yeast Co., Ltd.), Lipase (manufactured by Toyo Jozo Co. Ltd.), Lipex, Lipolase and Lipase SP-225 (which are manufactured by Novozymes A/S), Lipase (manufactured by Gist-Brocades International B.V.) and Lipase A and Lipase B (which are manufactured by Sapporo Breweries Ltd.) and preferably Lipex or Lipolase.

<37>

The method for accelerating an enzyme reaction according to any of <34> to <36>, wherein the protease is one or more selected from, besides an alkaline protease described in WO-A 99/018218 in which an amino acid sequence shown in Sequence No. 1 or 2 is preferably conserved in an amount of 70% or more or an alkaline protease described in JP-A H5-25492 which is preferably alkaline protease K-16 or alkaline protease K-14, the proteases produced by *Bacillus subtilis* sold under the trade names of Savinase®, Kannase®, Everlase®, Alcalase®, Polarzyme® and Esperase® manufactured by Novozymes A/S, the proteases supplied under the trade names of FN2®, FN3®, FN4®, Purafect® and Purafect Prime® manufactured by DuPont de Nemours, Inc. or variants of these and more preferably one or more selected from an enzyme described in WO-A 99/018218 in which an amino acid sequence shown in Sequence No. 1 or 2 is conserved in an amount of 80% or more, Savinase, Everlase, Alcalase and Progress

manufactured by Novozymes A/S (manufactured by Novozymes A/S) and Purafect and Purafect Prime manufactured by DuPont de Nemours, Inc.

<38>

The method for accelerating an enzyme reaction according to any of <34> to <37>, wherein the amylase is one or more amylases obtained from bacteria such as *Bacillus subtilis* Marburg, *Bacillus subtilis* natto, *Bacillus amyloliquefaciens, Bacillus licheniformis, Bacillus cereus, Bacillus macerans, Pseudomonas stutzeri, Klebsiella aerogenes* or the like, actinomycetes such as *Streptomyces griseus* or the like, fungi such as *Aspergillus oryzae, Aspergillus niger* or the like, seeds of gramineous and leguminous plants and digestive glands of animals such as humans and pigs or the like, preferably $\alpha$-amylase and more preferably one or more selected from the brand name Rapidase (manufactured by Gist-Brocades International B.V.), the brand names Termamyl, Duramyl and Stainzyme (manufactured by Novozymes Japan Ltd.), Amplify (manufactured by Novozymes A/S) and the brand names Purastar ST and Purastar OxAm (manufactured by Genencor International, Inc.).

<39>

The method for accelerating an enzyme reaction according to any of <1> to <38>, wherein the substrate is one or more selected from protein, starch and lipid.

<40>

The method for accelerating an enzyme reaction according to any of <1> to <39>, wherein the substrate is two or more substrates and further two or more substrates selected from protein, starch and lipid, and the method includes one or more of (1) reacting, using two or more enzymes, the substrate with the enzymes separately, (2) reacting the substrate with two or more enzymes combined into the state of a complex enzyme and (3) allowing multiple enzymes with different reactivity toward the substrate to act on the substrate in a stepwise manner.

<41>

The method for accelerating an enzyme reaction according to any of <1> to <40>, wherein the substrate is present in soil adhering to an article.

<42>

The method for accelerating an enzyme reaction according to any of <1> to <41>, wherein the substrate is present in soil adhering to a hard surface of an article having the hard surface.

<43>

The method for accelerating an enzyme reaction according to any of <1> to <41>, wherein the substrate is present in soil adhering to fibers.

<44>

An enzyme reaction accelerating agent containing, (a) a sulfosuccinic acid branched alkyl ester having a branched alkyl group with 9 or more and 12 or less carbons or a salt thereof as an active component.

<45>

An enzyme reaction accelerating agent composition containing, an enzyme reaction accelerating agent containing (a) a sulfosuccinic acid branched alkyl ester having a branched alkyl group with 9 or more and 12 or less carbons or a salt thereof [hereinafter referred to as component (a)] as an active component, and optionally, a surfactant other than component (a) [hereinafter referred to as component (b)], wherein when the composition contains component (b), a mass ratio of a content of component (a) to a content of component (b), (a)/(b), is 0.001 or more and 50 or less.

<46>

Use as an enzyme reaction accelerating agent of (a) a sulfosuccinic acid branched alkyl ester having a branched alkyl group with 9 or more and 12 or less carbons or a salt thereof.

<47>

A detergent composition for use in a dishwasher containing, (a) a sulfosuccinic acid branched alkyl ester having a branched alkyl group with 9 or more and 12 or less carbons [hereinafter referred to as component (a)], and (d) an enzyme [hereinafter referred to as component (d)].

<48>

The detergent composition for use in a dishwasher according to <47>, wherein the branched alkyl group of component (a) has a main chain with 6 or 7 carbons and one or more side chains, the side chains having 2 or more and 4 or less carbons in total.

<49>

The detergent composition for use in a dishwasher according to <47> or <48>, wherein the branched alkyl group of component (a) is one or more branched alkyl groups selected from 2-propylheptyl group and 3,5,5-trimethylhexyl group.

<50>

The detergent composition for use in a dishwasher according to any of <47> to <49>, wherein component (a) is a sulfosuccinic acid branched alkyl ester represented by the following formula 1:

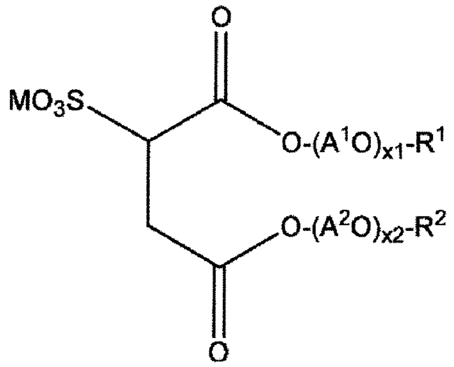

Formula 1

wherein $R^1$ and $R^2$ each represent a branched alkyl group with 9 or more and 12 or less carbons, $A^1O$ and $A^2O$ each represent an alkyleneoxy group with 2 or more and 4 or less carbons, x1 and x2 each represent an average number of added moles, which is a number of 0 or more and 10 or less, and M is a cation.

<51>

The detergent composition for use in a dishwasher according to <50>, wherein $R^1$ and $R^2$ in the formula 1 each have 1 or more, and preferably 3 or less and more preferably 2 or less side chains.

<52>

The detergent composition for use in a dishwasher according to <50> or <51>, wherein the open-chain branched hydrocarbon groups of $R^1$ and $R^2$ in the formula 1 each independently have 9 or 10 carbons in total, each independently have a main chain with 6 or 7 carbons, each independently have a side chain with 1 or more and 3 or less constituent carbons and each independently have one side chain.

<53>

The detergent composition for use in a dishwasher according to any of <50> to <52>, wherein the branched alkyl groups of $R^1$ and $R^2$ in the formula 1 are the same or different groups selected from 2-propylheptyl group and 3,5,5-trimethylhexyl group.

<54>

The detergent composition for use in a dishwasher according to any of <50> to <53>, wherein the average numbers of added moles of x and y in the formula 1 are each independently 0.

<55>

The detergent composition for use in a dishwasher according to any of <50> to <54>, wherein M in the formula 1 is an inorganic cation.

<56>

The detergent composition for use in a dishwasher according to any of <47> to <55>, containing component (a) in an amount of 0.01 mass% or more and 5 mass% or less.

<57>

The detergent composition for use in a dishwasher according to any of <47> to <56>, wherein component (d) is one or more selected from amylase, protease and lipase.

<58>

The detergent composition for use in a dishwasher according to any of <47> to <57>, wherein component (d) is preferably two or more enzymes selected from amylase, protease and lipase and two or more enzymes including at least amylase and protease.

<59>

The detergent composition for use in a dishwasher according to any of <47> to <58>, containing component (a) in an amount of 0.05 mass% or more and preferably 0.1 mass% or more, and 3 mass% or less, preferably 2 mass% or less and further preferably 1 mass% or less.

<60>

The detergent composition for use in a dishwasher according to any of <47> to <59>, containing component (d) in an amount of preferably 0.001 mass% or more, more preferably 0.005 mass% or more and further preferably 0.01 mass% or more, and preferably 15 mass% or less, more preferably 9 mass% or less and further preferably 5 mass% or less in terms of enzyme protein.

<61>

The detergent composition for use in a dishwasher according to any of <47> to <60>, wherein a mass ratio of a content of component (a) to a content of component (d), (a)/(d), is preferably 0.1 or more, more preferably 1 or more and further preferably 10 or more, and preferably 50 or less, more preferably 40 or less and further preferably 30

or less.

<62>

The detergent composition for use in a dishwasher according to any of <47> to <61>, further containing (b4) a nonionic surfactant [hereinafter referred to as component (b4)].

<63>

The detergent composition for use in a dishwasher according to <62>, wherein component (b4) is one or more nonionic surfactants selected from an alkyl glycoside, an alkyl glyceryl ether and a polyoxyalkylene alkyl ether.

<64>

The detergent composition for use in a dishwasher according to <62> or <63>, wherein component (b4) preferably has an alkyl group derived from a secondary alcohol and is a polyoxyalkylene alkyl ether with an oxyethylene group added to a secondary alcohol at an average number of added moles of 1 or more and 10 or less.

<65>

The detergent composition for use in a dishwasher according to any of <63> to <64>, containing component (b4) in an amount of preferably 0.05 mass% or more, more preferably 0.1 mass% or more and further preferably 0.3 mass% or more, and preferably 5 mass% or less, more preferably 3 mass% or less and further preferably 1.0 mass% or less.

<66>

The detergent composition for use in a dishwasher according to any of <47> to <65>, further containing one or more anti-foaming agents selected from silicone oil, polypropylene glycol and hydrophobic silica particles.

<67>

The detergent composition for use in a dishwasher according to <66>, wherein the weight average molecular weight of polypropylene glycol of an anti-foaming agent is preferably 1,000 or more, more preferably 2,000 or more and further preferably 3,000 or more, and preferably 10,000 or less, more preferably 8,000 or less and further preferably 5,000 or less.

<68>

The detergent composition for use in a dishwasher according to any of <47> to <67>, wherein a proportion of a content of component (a) in all the surfactants contained in the detergent composition for use in a dishwasher is 5 mass% or more, further 8 mass% or more and further 10 mass% or more, and 100 mass% or less, further 50 mass% or less and further 30 mass% or less.

<69>

The detergent composition for use in a dishwasher according to any of <47> to <68>, wherein the detergent composition for use in a dishwasher of the present invention optionally contains an anionic surfactant other than component (a), and a proportion of a content of component (a) in all the anionic surfactants is preferably 50 mass% or more and more preferably 90 mass% or more, and 100 mass% or less.

<70>

The detergent composition for use in a dishwasher according to any of <47> to <69>, further containing one or more chelating agents selected from citric acid, glutamic acid diacetic acid and salts of these.

<71>

The detergent composition for use in a dishwasher according to any of <47> to <70>, wherein the detergent composition for use in a dishwasher is liquid, and the composition has a pH of preferably 5 or more, more preferably 6 or more and further preferably 7 or more, and preferably 11 or less, more preferably 10 or less and further preferably 8 or less at 25°C.

<72>

The detergent composition for use in a dishwasher according to any of <47> to <71>, wherein the detergent composition for use in a dishwasher is liquid, and the composition has a viscosity of preferably 10 mPa·s or more, more preferably 50 mPa·s or more and further preferably 100 mPa·s or more, and preferably 5,000 mPa·s or less and more preferably 2,500 mPa·s or less at 25°C.

<73>

The detergent composition for use in a dishwasher according to any of <47> to <72>, wherein the detergent composition for use in a dishwasher is powder, the composition containing an alkali agent, and the alkali agent is one or more selected from sodium carbonate, potassium carbonate, sodium hydrogencarbonate, ethanolamine, sodium silicate and potassium silicate.

<74>

The detergent composition for use in a dishwasher according to any of <47> to <73>, wherein the concentration of the detergent composition for use in a dishwasher at the time of use (dilution concentration) is preferably 0.01 mass% or more, more preferably 0.05 mass% or more and further preferably 0.1 mass% or more, and preferably 2 mass% or less, more preferably 1 mass% or less and further preferably 0.5 mass% or less.

<75>

The detergent composition for use in a dishwasher according to any of <47> to <74>, wherein the concentration of component (a) at the time of use of the detergent composition for use in a dishwasher is preferably 1 ppm or more, more preferably 2 ppm or more and further preferably 5 ppm or more, and preferably 100 ppm or less, more preferably 50 ppm or less and further preferably 30 ppm or less.

<76>

The detergent composition for use in a dishwasher according to any of <47> to <75>, wherein the concentration of component (d) at the time of use of the detergent composition for use in a dishwasher is preferably 0.001 ppm or more, more preferably 0.005 ppm or more and further preferably 0.01 ppm or more, and preferably 5 ppm or less, more preferably 2 ppm or less and further preferably 1 ppm or less.

<77>

The detergent composition for use in a dishwasher according to any of <47> to <76>, wherein a mixture of the detergent composition for use in a dishwasher in an amount of 0.2 mass% and water has a pH of preferably 5 or more, more preferably 6 or more and further preferably 6.5 or more, and preferably 12 or less, more preferably 11.5 or less and further preferably 11 or less at 25°C.

<78>

A method for washing tableware including, washing the tableware in a dishwasher with the detergent composition for use in a dishwasher according to any of <47> to <77>.

<79>

The method for washing tableware according to <78>, wherein the tableware is washed in the dishwasher with a washing liquid prepared by diluting the detergent composition with water.

<80>

The method for washing tableware according to <78> or <79>, wherein the washing liquid has a washing temperature of preferably 30°C or more and more preferably 40°C or more, and preferably 80°C or less and more preferably 70°C or less.

<81>

Use as a detergent for use in a dishwasher of the composition according to any of <47> to <77>.

Examples

<Example 1a and comparative example 1a>

Triglyceride at triolein/tristearin = 8.5/1.5 (mass ratio) was dissolved in chloroform to prepare model soil. A polypropylene test piece with 75 mm (width) × 100 mm (length) × 1 mm (thickness) was coated uniformly with the model soil such that the coating amount was 0.1 g, and chloroform was evaporated and dried, thus preparing a soil piece.

An aqueous medium in Table 1 was placed in a beaker, and the soil piece was immersed therein such that the entire portion coated with the model soil was brought into contact with the aqueous medium. The aqueous medium had a temperature of 25°C.

After 10 minutes of immersion, hydrochloric acid was added to deactivate the enzyme.

The soil piece was taken out, and rinsed under running distilled water for 15 seconds. At that time, the whole amount of water during rinsing was collected. After the completion of rinsing, soil remaining on the soil piece was washed away with 50 ml of chloroform, which was placed in a separatory funnel together with the water collected during rinsing, and after several times of shaking, the lower chloroform layer was collected. 25 ml of chloroform was then added again, and after several times of shaking again, the chloroform layer was collected. After this operation was repeated three times, the collected chloroform was transferred to an eggplant flask, and chloroform was evaporated with an evaporator. After 10 ml of methanol was then added to the eggplant flask to dissolve the extract therein, the triglyceride amount (the total amount of triolein and tristearin) after immersion was quantified by LC/MS. Triglyceride decomposition rate (I) was determined by the formula blow from the triglyceride amounts before and after immersion. The results are shown in Table 1. A higher triglyceride decomposition rate (I) means that the enzyme reaction is more accelerated.

Triglyceride decomposition rate (I) (%) = (total amount of triglyceride before immersion - total amount of triglyceride after immersion)/total amount of triglyceride before immersion × 100

[Table 1]

| | | | Example | | | | | | | | | Comparative example | | | | | | | | | |
|---|---|---|---|---|---|---|---|---|---|---|---|---|---|---|---|---|---|---|---|---|---|
| | | | 1a-1 | 1a-2 | 1a-3 | 1a-4 | 1a-5 | 1a-6 | 1a-7 | 1a-8 | 1a-9 | 1a-1 | 1a-2 | 1a-3 | 1a-4 | 1a-5 | 1a-6 | 1a-7 | 1a-8 | 1a-9 | 1a-10 |
| Aqueous medium | Formulation component (mass%) | (a) a-1 | 0.2 | 0.05 | 0.1 | 1 | 5 | 2 | 5 | 0.005 | 0.01 | | | | | | | | | | |
| | | (a') a'-1 | | | | | | | | | | 1 | 5 | | | | | | | | |
| | | (b) LAS | | | | | | | | | | | | 0.2 | | | | 2 | | | |
| | | (b) AS | | | | | | | | | | | | | 0.2 | | | | 2 | | |
| | | (b) FA | | | | | | | | | | | | | | 0.2 | | | | 2 | |
| | | (b) AG | | | | | | | | | | | | | | | 0.2 | | | | 2 |
| | | (b) Betaine | | 0.2 | 0.2 | 0.2 | 0.2 | | | | | 0.8 | | 0.2 | 0.2 | 0.2 | 0.2 | 2 | 2 | 2 | 2 |
| | | (b) Amine oxide (1) | | | | | | 0.05 | | | 0.5 | | | | | | | | | | |
| | | (b) Amine oxide (2) | | | | | | | 1 | 5 | | | | | | | | | | | |
| | | Butyldiglycol | 6.5 | 6.5 | 6.5 | 6.5 | 6.5 | 6.5 | 6.5 | 6.5 | 6.5 | 6.5 | 6.5 | 6.5 | 6.5 | 6.5 | 6.5 | 6.5 | 6.5 | 6.5 | 6.5 |
| | | Lipase | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 |
| | | Water | Balance | Balance | Balance | Balance | Balance | Balance | Balance | Balance | Balance | Balance | Balance | Balance | Balance | Balance | Balance | Balance | Balance | Balance | Balance |
| | | Total | 100 | 100 | 100 | 100 | 100 | 100 | 100 | 100 | 100 | 100 | 100 | 100 | 100 | 100 | 100 | 100 | 100 | 100 | 100 |
| Triglyceride decomposition rate (I) (%) | | | 2.9 | 2.9 | 3.0 | 2.4 | 0.8 | 1.5 | 0.6 | 0.6 | 1.8 | 0.5 | 0.2 | 0.05 | 0.07 | 0.02 | 0.04 | 0.03 | 0.01 | 0.01 | 0.01 |

The components in the table are the following. Note that mass% of each component in the table is based on the amount in terms of an active component (the same applies hereinafter). Component (a') is a comparative component for component (a).

· a-1: sodium bis-(2-propylheptyl)sulfosuccinate
· a'-1: sodium bis-(2-ethylhexyl)sulfosuccinate
· LAS: Sodium Dodecylbenzene Sulfonate manufactured by FUJIFILM Wako Pure Chemical Corporation
· AS: Sodium Dodecyl Sulfate manufactured by FUJIFILM Wako Pure Chemical Corporation
· FA: Sodium Laurate manufactured by FUJIFILM Wako Pure Chemical Corporation
· AG: product name "PLANTACARE 2000 UP" manufactured by BASF SE, an alkyl polyglucoside in which an alkyl group has 8 or more and 16 or less carbons and a degree of condensation of glucose is 1 or more and 2 or less
· Betaine: laurylhydoxysulfo betaine, AMPHITOL 20HD manufactured by Kao Corporation
· Amine oxide (1): lauryldimethylamine oxide, AMPHITOL 20N manufactured by Kao Corporation
· Amine oxide (2): octyldimethylamine oxide, AMPHITOL 08N manufactured by Kao Corporation
· Lipase: product name "Lipex Evity 100L" manufactured by Novozymes A/S with a molecular weight of about 30000

<Example 2a>

An aqueous medium in Table 2 was filled into a pressure-accumulating-type foam-discharge trigger-type spray container (CuCute Clear Foam Spray manufactured by Kao Corporation), and sprayed five times on a soil piece prepared in the same manner as in example 1a. The aqueous medium was discharged in the form of foam. The discharge amount of the aqueous medium was about 3 g in total. Each component in Table 2 is the same as that used in example 1a.

After brought into contact with the discharged foam for 1 minute, the soil piece was rinsed under running distilled water for 15 seconds. At that time, the ambient temperature was 25°C. Further, the whole amount of water during rinsing was collected. After the completion of rinsing, soil remaining on the plate was washed away with 50 ml of chloroform, which was placed in a separatory funnel together with the water collected during rinsing, and after several times of shaking, the lower chloroform layer was collected. 25 ml of chloroform was then added again, and after several times of shaking again, the chloroform layer was collected. After this operation was repeated three times, the collected chloroform was transferred to an eggplant flask, and chloroform was evaporated with an evaporator. After 10 ml of 2-propanol was then added to the eggplant flask to dissolve the extract therein, the amount of each component, triolein (a), tristearin (b), diolein (c), distearin (d), monoolein (e), monostearin (f), oleic acid (g), stearic acid (h) or glycerin (i) was quantified from peak areas by LC/MS. Triglyceride decomposition rate (II) was determined by the formula below from the amount of each component. The results are shown in Table 2. A higher triglyceride decomposition rate (II) means that the enzyme reaction is more accelerated.

Triglyceride decomposition rate (II) (%) = {(c) + (d) + (e) + (f) + (g) + (h) + (i)}/{(a) + (b) + (c) + (d) + (e) + (f) + (g) + (h) + (i)} × 100

[Table 2]

| | | | | Example | | | | | | | |
|---|---|---|---|---|---|---|---|---|---|---|---|
| | | | | 2a-1 | | | | 2a-2 | | | |
| | | | | a | b | c | d | a | b | c | d |
| Aqueous medium | Formulation component (mass%) | (a) | a-1 | 0.2 | 0.2 | 0.2 | 0.2 | 2 | 2 | 2 | 2 |
| | | (b) | Betaine | | 0.2 | | | | 2 | | |
| | | | Amine oxide (1) | | | 0.2 | | | | 2 | |
| | | | Amine oxide (2) | | | | 0.2 | | | | 2 |
| | | Butyldiglycol | | 6.5 | 6.5 | 6.5 | 6.5 | 6.5 | 6.5 | 6.5 | 6.5 |
| | | Lipase | | 0.05 | 0.05 | 0.05 | 0.05 | 0.05 | 0.05 | 0.05 | 0.05 |
| | | Water | | Balance | Balance | Balance | Balance | Balance | Balance | Balance | Balance |
| | | Total | | 100 | 100 | 100 | 100 | 100 | 100 | 100 | 100 |
| Triglyceride decomposition rate (II) (%) | | | | 1.2 | 1.9 | 2.1 | 2.2 | 0.9 | 1.0 | 1.3 | 1.3 |

Table 2 shows that triglyceride decomposition rate (II) is increased in an example using an aqueous medium in which component (b) is used in combination. This is considered to be because the quality of foam formed from aqueous media differs depending on the presence or absence of component (b). In other words, it is inferred that as foam created from an aqueous medium in which component (b) is used in combination has finer texture and higher ability to retain its foam state than foam created from an aqueous medium in which component (b) is not used in combination, a contact opportunity between the substrate and the enzyme whose activity is enhanced by component (a) increases, resulting in a higher triglyceride decomposition rate (II).

<Example 3a and comparative example 3a>

(1) Formulation component

The components used in example 3a (examples 3a-1 to 3a-10) and comparative example 3a (comparative examples 3a-1 to 3a-10) are the following. Component (a') is a comparative component for component (a).

[Component (a)]

· a-1: sodium bis-(2-propylheptyl)sulfosuccinate

[Component (a')]

· a'-1: sodium bis-(2-ethylhexyl)sulfosuccinate
· AS: Sodium Dodecyl Sulfate manufactured by FUJIFILM Wako Pure Chemical Corporation

[Enzyme]

· Protease: product name "Savinase 16L" manufactured by Novozymes A/S

[Other components]

· A tris hydrochloric acid buffer (manufactured by NIPPON GENE CO., LTD.)
· Distilled Water (manufactured by FUJIFILM Wako Pure Chemical Corporation)

(1) Method for evaluating protease activity

BSA (bovine serum albumin; FUJIFILM Wako Pure Chemical Corporation) was added as a protein substrate to 1 mL of each aqueous medium shown in Table 3 such that the concentration was 50 ppm, stirred with a vortex mixer for 3 seconds, and then incubated at 25°C. An aqueous medium in Table 3 was produced by mixing each component with the above distilled water, and appropriately with the tris hydrochloric acid buffer (manufactured by NIPPON GENE CO., LTD.) to adjust the pH to the value in the table. 30 minutes later, the reaction solution was mixed at 1:1 with 2 × Ez-Apply sample buffer (manufactured by ATTO Corporation) containing 10 mM phenylmethylsulfonyl fluoride, stirred with a vortex mixer for 3 seconds, and then incubated at 100°C for 5 minutes.

Using e-PAGEL e-T1020L (manufactured by ATTO Corporation) as an electrophoresis gel and XL-Ladder Broad SP-2110 (manufactured by Apro Science Corporation) as a molecular weight marker, electrophoresis was carried out at 40 mA for 80 minutes (WSE-1100 PageRun-R (manufactured by ATTO Corporation)). One-step Ruby (manufactured by Apro Science Corporation) was used for dyeing the gel.

Fluorescence images of the gel were taken, and band intensities in the 50-80 kDa region were measured from the acquired images using image analysis software (manufactured by Bio-Rad Laboratories, Inc.). BSA decomposition rate was calculated by the formula below as an indicator of enzyme activity, and the BSA decomposition rate value was evaluated in the following manner. The results are shown in Table 3. In this evaluation, a BSA decomposition rate of 30% or more is desirable, and a higher value is more desirable.

·

BSA decomposition rate (%) = 100 - (band intensity after decomposition/band intensity in undecomposed state) × 100

[Table 3]

| | | | Example | | | | | | | | | |
|---|---|---|---|---|---|---|---|---|---|---|---|---|
| | | | 3a-1 | 3a-2 | 3a-3 | 3a-4 | 3a-5 | 3a-6 | 3a-7 | 3a-8 | 3a-9 | 3a-10 |
| Aqueous medium | Formulation component | (a) a-1 (ppm) | 50 | 100 | 250 | 500 | 1000 | 50 | 100 | 250 | 500 | 1000 |
| | | (a') a'-1 (ppm) | | | | | | | | | | |
| | | (a') AS (ppm) | | | | | | | | | | |
| | | Protease (ppm) | 1.5 | 1.5 | 1.5 | 1.5 | 1.5 | 1.5 | 1.5 | 1.5 | 1.5 | 1.5 |
| | Hardness (°DH) | | 0 | 0 | 0 | 0 | 0 | 4 | 4 | 4 | 4 | 4 |
| pH (20°C) | | | 7.5 | 7.5 | 7.5 | 7.5 | 7.5 | 7.5 | 7.5 | 7.5 | 7.5 | 7.5 |
| Substrate | Bovine serum albumin (ppm) | | 50 | 50 | 50 | 50 | 50 | 50 | 50 | 50 | 50 | 50 |
| BSA decomposition rate (%) | | | 37.9 | 61.2 | 93.5 | 85.1 | 84.3 | 31.2 | 55.8 | 95.9 | 91.3 | 92.2 |

| | | | Comparative example | | | | | | | | | |
|---|---|---|---|---|---|---|---|---|---|---|---|---|
| | | | 3a-1 | 3a-2 | 3a-3 | 3a-4 | 3a-5 | 3a-6 | 3a-7 | 3a-8 | 3a-9 | 3a-10 |
| Aqueous medium | Formulation component | (a) a-1 (ppm) | 1000 | | | | | | | | | |
| | | (a') a'-1 (ppm) | | | | 50 | 100 | 250 | | | | |
| | | (a') AS (ppm) | | | | | | | 50 | 100 | 250 | 500 |
| | | Protease (ppm) | | 1.5 | 1.5 | 1.5 | 1.5 | 1.5 | 1.5 | 1.5 | 1.5 | 1.5 |
| | Hardness (°DH) | | 0 | 0 | 4 | 0 | 0 | 0 | 0 | 0 | 0 | 0 |
| pH (20°C) | | | 7.5 | 7.5 | 7.5 | 7.5 | 7.5 | 7.5 | 7.5 | 7.5 | 7.5 | 7.5 |
| Substrate | Bovine serum albumin (ppm) | | 50 | 50 | 50 | 50 | 50 | 50 | 50 | 50 | 50 | 50 |
| BSA decomposition rate (%) | | | 0.0 | 0.0 | 0.0 | 0.0 | 0.0 | 21.7 | 0.0 | 0.0 | 0.0 | 10.5 |

In the table, the hardness of an aqueous medium is expressed in terms of German hardness (°dH) measured by the method below. The table shows a hardness adjusted by diluting an aqueous medium with a mother liquid aqueous solution having a hardness of 4000°DH prepared by mixing 1 L of water, 83.9 g of calcium chloride dihydrate and 29.0 g of magnesium chloride hexahydrate such that the aqueous medium had a required hardness.

German hardness (°dH) in the present specification refers to the concentration of calcium and magnesium in water expressed in terms of a $CaCO_3$ concentration with 1 mg/L (ppm) = about 0.056°dH (1°dH = 17.8 ppm). This concentration of calcium and magnesium for German hardness is determined by a chelate titration method using ethylenediaminetetraacetic acid disodium salt. A specific method for measuring the German hardness of an aqueous medium in the present specification is described below.

<Method for measuring German hardness of aqueous medium>

[Reagent]

· 0.01 mol/l EDTA.2Na solution: a 0.01 mol/l ethylenediaminetetraacetic acid disodium salt solution (a solution for titration, 0.01 M EDTA-Na2 manufactured by Sigma-Aldrich)
· Indicator Universal BT (product name: Universal BT manufactured by DOJINDO LABORATORIES)
· Ammonia buffer solution for hardness measurements (a solution obtained by dissolving 67.5 g of ammonium chloride in 570 ml of 28 w/v% ammonia water and making the total amount 1000 ml with ion exchange water)

[Measurement of hardness]

(1) 20 ml of an aqueous medium as a sample was collected in a conical beaker with a volumetric pipette.
(2) 2 ml of ammonia buffer solution for hardness measurements was added.
(3) 0.5 ml of indicator Universal BT was added. It was confirmed that the solution after the addition was reddish purple.
(4) While the conical beaker was shaken well, 0.01 mol/l EDTA.2Na solution was added dropwise thereto from a burette, and the point of time when the sample aqueous medium turned blue was taken as the end point of titration.
(5) The total hardness was determined by the following calculation formula:

$$\text{Hardness (°dH)} = T \times 0.01 \times F \times 56.0774 \times 100/A$$

T: titration amount of 0.01 mol/l EDTA·2Na solution (mL)
A: sample volume (20 mL, the volume of a sample aqueous medium)
F: factor for 0.01 mol/l EDTA·2Na solution

<Examples 1b and 2b and comparative examples 1b and 2b>

In examples 1b and 2b and comparative examples 1b and 2b, detergent compositions for use in a dishwasher containing the following components (a), (d) and (b4) or the like at proportions in Tables 4 and 5 were prepared, and evaluations of rice soil washing performance, protein washing performance (finishing performance) and oil soil washing performance were conducted on each of them.

<Component (a)>

· Sodium di-(2-propylheptyl)sulfosuccinate

<Component (a')>

· Sodium di-(2-ethylhexyl)sulfosuccinate: SANMORIN OT-70 (manufactured by Sanyo Chemical Industries, Ltd.)

<Component (d): enzyme>

Amylase: Duramyl 120T (manufactured by Novozymes A/S)
Protease: Savinase 18T-B (manufactured by Novozymes A/S)

<Component (b4)>

· A polyoxyethylene alkyl ether (in which the average number of added moles of EO is 5 and an alkyl group has 12 to 14 carbons), SOFTANOL 50 (manufactured by NIPPON SHOKUBAI CO., LTD.)

<Other components>

· A sodium polyoxypropylene alkyl ether sulfate (in which the average number of added moles of PO is 0.6 and an alkyl group has 8 to 12 carbons)
· Polypropylene glycol: Polypropylene Glycol (with a weight average molecular weight of 3000) (manufactured by Wako Pure Chemical Industries, Ltd.)
· Chelating agent: Citric Acid Hydrate (manufactured by Wako Pure Chemical Industries, Ltd.)
· Chelating agent: trisodium citrate, refined sodium citrate (manufactured by FUSO CHEMICAL CO., LTD.)
· Chelating agent: GLDA, tetrasodium L-glutamate diacetate (manufactured by Nouryon N.V.)
· Thickener: Xanthan Gum (manufactured by Tokyo Chemical Industry Co., Ltd.)
· Alkali agent: sodium carbonate, Soda Ash (dense) (manufactured by Tokuyama Corporation)
· Sodium sulfate: Sodium Sulfate Decahydrate (manufactured by FUJIFILM Wako Pure Chemical Corporation)
· Water

<Method for evaluating washing performance>

(1) Method for evaluating rice soil washing performance

A ceramic rice bowl was coated with 0.5 g of rice soil and dried overnight to prepare a soiled rice bowl. Two soiled rice bowls were placed within an automatic dishwasher (model number NP-45MS8S manufactured by Panasonic Corporation) with unsoiled rice bowls of the same type on the inside to prevent the soiled rice bowls from coming in direct contact with water. 6 g of a detergent composition for use in a dishwasher in the tables was put thereinto, and washing was carried out with the standard course. After washing, remaining soil was dyed with iodine to be visible, and an evaluation score was then given on the basis of the following criteria.

Note that the concentration of component (a) in a washing liquid applied to the tableware (a washing liquid formed in such a manner that a detergent composition for use in a dishwasher was diluted with water) was 10 ppm when the put amount of the composition was 6 g.

* Evaluation criteria for rice soil washing performance
3: The soil has been removed cleanly.
2: Most of the soil has been removed, but some remains.
1: The soil remains, and most of the soil is visible.

(2) Method for evaluating protein washing performance (finishing performance)

(2-1) Method for preparing protein soil

After making an over easy fried egg (in the state where the egg yolk portion is hardened being half-fried) in a frying pan, only the egg liquid was collected with a strainer.

(2-2) Evaluation method

Two clean glass cups were placed within an automatic dishwasher, into which 10 g of the over easy egg collected in (2-1) and 6 g of a detergent composition for use in a dishwasher in the tables were put, and washing was carried out with the standard course. After washing, an evaluation score was given on the basis of the following criteria.

Note that the concentration of component (a) in a washing liquid applied to the tableware (a washing liquid formed in such a manner that a detergent composition for use in a dishwasher was diluted with water) was 10 ppm when the put amount of the composition was 6 g.

* Evaluation criteria for protein washing performance
3: There are about 10 water spots and little or no dullness.
2: There are about 20 to 50 water spots or about 25% of the entire portion is dull and white.
1: Half or more of the entire potion is dull.

(3) Method for evaluating oil soil washing performance

Four polypropylene dishes were placed within an automatic dishwasher. Two of the dishes were soiled dishes coated with complex soil at pork fat/rapeseed oil = 1/1 (mass ratio) in an amount of 3 g per dish, and the remaining two were unsoiled dishes.

6 g of a detergent composition for use in a dishwasher in the tables was put thereinto, and washing was carried out with the standard course. A total of three panelists touched the dishes after washing and gave evaluation scores on the basis of the following criteria. Tables 4 and 5 show the averages of the evaluation scores rounded to one decimal place.

Note that the concentration of component (a) in a washing liquid applied to the tableware (a washing liquid formed in such a manner that a detergent composition for use in a dishwasher was diluted with water) was 10 ppm when the put amount of the composition was 6 g.

* Evaluation criteria for oil soil washing performance
4: The soil has been removed, and there is a squeaky and pleasant feeling to the touch.
3: The soil has been removed, but there is no squeaky feeling to the touch.
2: Most of the soil seems to have been washed, but streaks appear when a dish was rubbed with a finger.
1: It can be seen that oil remains.

<pH measurement method>

The pH at 25°C of a washing liquid prepared by diluting each of the detergent compositions for use in a dishwasher of the examples and comparative examples with water such that the concentration was 0.2 mass% was measured by a glass electrode method. The results are shown in Tables 4 and 5.

<Viscosity measurement method>

The viscosity at 25°C of each of the detergent compositions for use in a dishwasher of the examples and comparative examples in Table 5 was measured with a B-type viscometer (rotor No. 2, 6 rpm, measurement time 1 minute, liquid temperature 25°C). The results are shown in Table 5.

[Table 4]

| | | | | Example | Comparative example | | |
|---|---|---|---|---|---|---|---|
| | | | | 1b-1 | 1b-1 | 1b-2 | 1b-3 |
| Detergent composition for use in dishwasher | Formulation amount (mass%) | (a) | Sodium di-(2-propylheptyl) sulfosuccinate | 0.5 | | 0.5 | |
| | | (b4) | Polyoxyethylene alkyl ether | | | | 0.5 |
| | | Polypropylene glycol | | 2.5 | 2.5 | 2.5 | 2.5 |
| | | Trisodium citrate | | 15 | 15 | 15 | 15 |
| | | Sodium carbonate | | 25 | 25 | 25 | 25 |
| | | (d) | Amylase | 0.02 | 0.02 | | 0.02 |
| | | | Protease | 0.02 | 0.02 | | 0.02 |
| | | Sodium sulfate | | Balance | Balance | Balance | Balance |
| | | Total | | 100 | 100 | 100 | 100 |
| | pH of diluted liquid (composition concentration 0.2 mass%) (25°C) | | | 11 | 11 | 11 | 11 |
| Washing performance | | Rice soil washing performance | | 3 | 2 | 1 | 2 |
| | | Oil soil washing performance | | 4 | 1 | 4 | 2 |
| | | Protein washing performance (finishing performance) | | 3 | 2 | 1 | 2 |

It is inferred from a comparison of example 1b-1 and comparative examples 1b-1 and 1b-3 in Table 4 that component (a) of the present invention more improves the decomposition reaction of a substrate (soil) with component (d). Note that high oil washing performance of the compositions of example 1b-1 and comparative example 1b-2 regardless of no formulation with an enzyme that acts on oil (lipase) in Table 4 is considered to be because of a high washing temperature of the automatic dishwasher and oil soil removal performance and dispersion effect based on the formulation with component (a) .

[Table 5]

| | | | | Example | | | Comparative example | | | | |
|---|---|---|---|---|---|---|---|---|---|---|---|
| | | | | 2b-1 | 2b-2 | 2b-3 | 2b-1 | 2b-2 | 2b-3 | 2b-4 | 2b-5 |
| Detergent composition for use in dishwasher | Formulation amount (mass%) | (a) | Sodium di-(2-propylheptyl) sulfosuccinate | 0.5 | 0.5 | 0.5 | 0.5 | | | | |
| | | (a') | Sodium di-(2-ethylhexyl) sulfosuccinate | | | | | 0.5 | | | |
| | | (d) | Amylase | 0.01 | 0.01 | 0.01 | | 0.01 | 0.01 | 0.01 | 0.01 |
| | | | Protease | 0.01 | 0.01 | 0.01 | | 0.01 | 0.01 | 0.01 | 0.01 |
| | | (b4) | Polyoxyethylene alkyl ether | | 0.5 | | | | 0.5 | | |
| | | Sodium polyoxypropylene (PO = 0.6) alkyl ether sulfate | | | | 5 | | | | | |
| | | Polypropylene glycol | | | | | | | | 0.5 | |
| | | Xanthan gum | | 0.4 | 0.4 | 0.4 | 0.4 | 0.4 | 0.4 | 0.4 | 0.4 |
| | | Citric acid hydrate | | 4 | 4 | 4 | 4 | 4 | 4 | 4 | 4 |
| | | GLDA | | 2 | 2 | 2 | 2 | 2 | 2 | 2 | 2 |
| | | Water | | Balance | Balance | Balance | Balance | Balance | Balance | Balance | Balance |
| | | Total | | 100 | 100 | 100 | 100 | 100 | 100 | 100 | 100 |
| | pH of diluted liquid (composition concentration 0.2 mass%) (25°C) | | | 7.5 | 7.5 | 7.5 | 7.5 | 7.5 | 7.5 | 7.5 | 7.5 |
| | Viscosity (25°C) (mPa·s) | | | 1800 | 1800 | 1800 | 1800 | 1800 | 1800 | 1800 | 1800 |
| Washing performance | | | Rice soil washing performance | 3 | 3 | 3 | 1 | 3 | 3 | 3 | 3 |
| | | | Oil soil washing performance | 4 | 4 | 4 | 4 | 2 | 1 | 1 | 1 |
| | | | Protein washing performance (finishing performance) | 3 | 3 | 3 | 1 | 2 | 2 | 2 | 2 |

It is inferred from a comparison of example 2b-1 and comparative examples 2b-2 and 2b-5 in Table 5 that component (a) of the present invention more improves the decomposition reaction of a substrate (soil) with component (d). Note that while there is no difference in rice soil washing performance between examples 2b-1 to 2b-3 and comparative example 2b-5 in this evaluation, this is because amylase used therein is relatively more likely to exhibit its performance in a liquid-state composition, resulting in no apparent difference in this evaluation. For example, if evaluations are made under more severe washing conditions (a situation where the substrate is less likely to be brought into contact with a washing liquid due to washing in a crammed dishwasher, a situation where the target is complex soil of rice and lipid, or the like), there may be a difference between these examples and comparative example. Further, high oil soil washing performance of the compositions of examples 2b-1 to 2b-3 and comparative example 2b-1 regardless of no formulation with an enzyme that acts on oil (lipase) in table 5 is considered to be because of a high washing temperature of the automatic dishwasher and oil soil removal performance and dispersion effect based on the formulation with component (a).

In Tables 4 and 5, the formulation amount of component (d) is expressed in terms of mass% of enzyme protein.

## Claims

1. A method for accelerating an enzyme reaction comprising, reacting a substrate with an enzyme in the presence of (a) a sulfosuccinic acid branched alkyl ester having a branched alkyl group with 9 or more and 12 or less carbons or a salt thereof [hereinafter referred to as component (a)].

2. The method for accelerating an enzyme reaction according to claim 1, wherein the component (a) is a sulfosuccinic acid branched alkyl ester having a branched alkyl group with 9 or 10 carbons or a salt thereof.

3. The method for accelerating an enzyme reaction according to claim 1 or 2, wherein an aqueous medium containing the component (a), the enzyme and water is brought into contact with the substrate to react the substrate with the enzyme.

4. The method for accelerating an enzyme reaction according to claim 3, wherein a content of the component (a) in the aqueous medium is 0.01 mass% or more and 1.0 mass% or less.

5. The method for accelerating an enzyme reaction according to claim 3 or 4, wherein the aqueous medium optionally comprises a surfactant other than the component (a) [hereinafter referred to as component (b)], and a content of the component (b) in the aqueous medium is 3.0 mass% or less.

6. The method for accelerating an enzyme reaction according to any one of claims 3 to 5, wherein the aqueous medium is brought into contact with the substrate in the state of foam.

7. The method for accelerating an enzyme reaction according to any one of claims 1 to 6, wherein the enzyme is one or more selected from protease, amylase and lipase.

8. The method for accelerating an enzyme reaction according to any one of claims 1 to 7, wherein the substrate is one or more selected from protein, starch and lipid.

9. The method for accelerating an enzyme reaction according to any one of claims 1 to 8, wherein the substrate is present in soil adhering to an article.

10. The method for accelerating an enzyme reaction according to any one of claims 1 to 9, wherein the substrate is present in soil adhering to a hard surface of an article having the hard surface.

11. The method for accelerating an enzyme reaction according to any one of claims 1 to 9, wherein the substrate is present in soil adhering to a fiber portion of an article including fibers.

12. An enzyme reaction accelerating agent comprising, (a) a sulfosuccinic acid branched alkyl ester having a branched alkyl group with 9 or more and 12 or less carbons or a salt thereof [hereinafter referred to as component (a)] as an active component.

13. An enzyme reaction accelerating agent composition comprising, an enzyme reaction accelerating agent containing (a) a sulfosuccinic acid branched alkyl ester having a branched alkyl group with 9 or more and 12 or less carbons or a salt thereof [hereinafter referred to as component (a)] as an active component, and optionally, a surfactant other than the component (a) [hereinafter referred to as component (b)], wherein when the composition contains the component (b), a mass ratio of a content of the component (a) to a content of the component (b), (a)/(b), is 0.001 or more and 50 or less.

14. Use as an enzyme reaction accelerating agent of (a) a sulfosuccinic acid branched alkyl ester having a branched alkyl group with 9 or more and 12 or less carbons or a salt thereof.

15. A detergent composition for use in a dishwasher comprising, (a) a sulfosuccinic acid branched alkyl ester having a branched alkyl group with 9 or more and 12 or less carbons or a salt thereof [hereinafter referred to as component (a)], and (d) an enzyme [hereinafter referred to as component (d)].

16. The detergent composition for use in a dishwasher according to claim 15, comprising the component (a) in an amount of 0.01 mass% or more and 5 mass% or less.

**17.** The detergent composition for use in a dishwasher according to claim 15 or 16, wherein the component (d) is one or more selected from amylase, protease and lipase.

**18.** The detergent composition for use in a dishwasher according to any one of claims 15 to 17, wherein the branched alkyl group of the component (a) is one or more branched alkyl groups selected from 2-propylheptyl group and 3,5,5-trimethylhexyl group.

**19.** The detergent composition for use in a dishwasher according to any one of claims 15 to 18, further comprising (b4) a nonionic surfactant.

**20.** The detergent composition for use in a dishwasher according to any one of claims 15 to 19, further comprising one or more chelating agents selected from citric acid, glutamic acid diacetic acid and salts of these.

**21.** The detergent composition for use in a dishwasher according to any one of claims 15 to 20, further comprising one or more anti-foaming agents selected from silicone oil, polypropylene glycol and hydrophobic silica particles.

**22.** The detergent composition for use in a dishwasher according to any one of claims 15 to 21, wherein the detergent composition for use in a dishwasher is liquid, and the detergent composition for use in a dishwasher has a viscosity of 10 mPa·s or more and 5,000 mPa·s or less at 25°C.

**23.** The detergent composition for use in a dishwasher according to any one of claims 15 to 22, wherein a mixture containing the detergent composition for use in a dishwasher in an amount of 0.2 mass% and water has a pH of 5 or more and 12 or less at 25°C.

**24.** The detergent composition for use in a dishwasher according to any one of claims 15 to 23, wherein a mass ratio of a content of the component (a) to a content of the component (d), (a)/(d), is 0.1 or more and 50 or less.

**25.** A method for washing tableware comprising, washing the tableware in a dishwasher with the detergent composition according to any one of claims 15 to 24.

**26.** The method for washing tableware according to claim 25, wherein the tableware is washed in the dishwasher with a washing liquid prepared by diluting the detergent composition with water.

**27.** The method for washing tableware according to claim 26, wherien the washing liquid has a temperature of 30°C or more and 80°C or less.

**28.** Use as a detergent for use in a dishwasher of the composition according to any one of claims 15 to 24.

INTERNATIONAL SEARCH REPORT

International application No. **PCT/JP2022/021663**

**A. CLASSIFICATION OF SUBJECT MATTER**

***C12N 9/50***(2006.01)i; ***C11D 1/28***(2006.01)i; ***C11D 1/66***(2006.01)i; ***C11D 3/386***(2006.01)i; ***C12N 9/20***(2006.01)i; ***C12N 9/26***(2006.01)i
FI: C12N9/50 ZNA; C11D3/386; C12N9/26 A; C12N9/20; C11D1/66; C11D1/28

According to International Patent Classification (IPC) or to both national classification and IPC

**B. FIELDS SEARCHED**

Minimum documentation searched (classification system followed by classification symbols)

C12N9/00-9/99; C11D1/00-19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 2012-75379 A (SAN NOPCO LTD.) 19 April 2012 (2012-04-19)<br>claims 1-5, paragraphs [0108]-[0111], [0139], [0151], [0189], examples 17, 18, 21, tables 1, 2 | 1-14<br><br>15-28 |
| X<br><br>Y | JP 2015-199941 A (KAO CORP.) 12 November 2015 (2015-11-12)<br>claims 1, 3, 5, paragraphs [0010], [0012], [0037], examples | 1-10, 12-14<br><br>15–28 |
| Y | JP 2016-60798 A (KAO CORP.) 25 April 2016 (2016-04-25)<br>claims 1-2, 6, paragraphs [0010], [0037], [0054], [0058], [0063] | 15-28 |
| Y | JP 2004-203990 A (LION CORP.) 22 July 2004 (2004-07-22)<br>claims 3-4, paragraphs [0007], [0012], [0020] | 15-28 |
| A | JP 2019-182911 A (KAO CORP.) 24 October 2019 (2019-10-24) | 1-28 |
| A | JP 2020-152757 A (LION CORP.) 24 September 2020 (2020-09-24) | 1-28 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 July 2022** | **26 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/021663**

**C. DOCUMENTS CONSIDERED TO BE RELEVANT**

| Category* | Citation of document, with indication, where appropriate, of the relevant passages | Relevant to claim No. |
|---|---|---|
| A | JP 2020-100693 A (KAO CORP.) 02 July 2020 (2020-07-02) | 1-28 |
| A | JP 2020-100745 A (LION CORP.) 02 July 2020 (2020-07-02) | 1-28 |
| A | JP 2008-133340 A (KAO CORP.) 12 June 2008 (2008-06-12) | 1-28 |

Form PCT/ISA/210 (second sheet) (January 2015)

**INTERNATIONAL SEARCH REPORT**
**Information on patent family members**

International application No.
**PCT/JP2022/021663**

| Patent document cited in search report | Publication date (day/month/year) | Patent family member(s) | Publication date (day/month/year) |
|---|---|---|---|
| JP 2012-75379 A | 19 April 2012 | (Family: none) | |
| JP 2015-199941 A | 12 November 2015 | (Family: none) | |
| JP 2016-60798 A | 25 April 2016 | (Family: none) | |
| JP 2004-203990 A | 22 July 2004 | (Family: none) | |
| JP 2019-182911 A | 24 October 2019 | (Family: none) | |
| JP 2020-152757 A | 24 September 2020 | (Family: none) | |
| JP 2020-100693 A | 02 July 2020 | (Family: none) | |
| JP 2020-100745 A | 02 July 2020 | (Family: none) | |
| JP 2008-133340 A | 12 June 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S637789 A
- JP 2012075378 A
- JP 2019182911 A
- JP 2020100745 A
- JP H472397 A
- JP 2006152287 A
- US 2028091 B
- JP S5824555 A
- WO 99018218 A
- JP H525492 A